(19) Europäisches Patentamt
European Patent Office
Office européen des brevets

(11) **EP 1 227 105 A1**

(12) **EUROPEAN PATENT APPLICATION**
published in accordance with Art. 158(3) EPC

(43) Date of publication:
**31.07.2002 Bulletin 2002/31**

(21) Application number: **00970212.7**

(22) Date of filing: **31.10.2000**

(51) Int Cl.[7]: **C07K 14/47**, C12N 15/12, C07K 16/18, A61K 38/17, G01N 33/15, G01N 33/50

(86) International application number:
**PCT/JP00/07635**

(87) International publication number:
**WO 01/32705 (10.05.2001 Gazette 2001/19)**

(84) Designated Contracting States:
**AT BE CH CY DE DK ES FI FR GB GR IE IT LI LU MC NL PT SE**
Designated Extension States:
**AL LT LV MK RO SI**

(30) Priority: **01.11.1999 JP 31163299**
**17.12.1999 JP 35872399**

(71) Applicant: **Takeda Chemical Industries, Ltd.**
**Osaka-shi, Osaka 541-0045 (JP)**

(72) Inventors:
- **HINUMA, Shuji**
  **Tsukuba-shi, Ibaraki 305-0821 (JP)**
- **KAWAMATA, Yuji**
  **-chome, Tsukuba-shi, Ibaraki 305-0035 (JP)**
- **FUKUSUMI, Shoji**
  **Tsukuba-shi, Ibaraki 305-0044 (JP)**
- **FUJII, Ryo**
  **Tsukuba-shi, Ibaraki 305-0821 (JP)**

(74) Representative: **Lewin, John Harvey**
**Takeda Patent Office,**
**11-12 Charles II Street**
**London SW1Y 4QU (GB)**

# (54) GHSR LIGAND POLYPEPTIDES AND DNAS THEREOF

(57) The present invention relates to polypeptides characterized by containing an amino acid sequence that is the same or substantially the same as the amino acid sequence represented by SEQ ID NO: 1, or amides, esters or salts thereof, which are useful for developing pharmaceuticals such as GH secretion controlling agents; DNAs encoding these polypeptides, or amides, esters or salts thereof; a method/kit for screening a compound capable of changing the binding properties of these polypeptides to GHSR; etc.

EP 1 227 105 A1

[Sequence Listing]

<110> Takeda Chemical Industries, Ltd.

<120> GHSR Ligand Polypeptide and its DNA

<130> 2664WOOP

<150> JP 11-311632

<151> 1999-11-01

<150> JP 11-358723

<151> 1999-12-17

<160> 25

<210> 1

<211> 17

<212> PRT

<213> Human, Bovine

<400> 1

Thr Val Phe Phe Lys Thr Leu Arg Asn His Trp Lys Lys Thr Thr Ala

1 5 10 15

Gly

17

<210> 2

<211> 51

<212> DNA

<213> Bovine

<400> 2

ACTGTGTTCT TTAAAACGCT TCGAAATCAT TGGAAGAAAA CGACAGCTGG G 51

<210> 3

<211> 420

<212> PRT

<213> Bovine

<400> 3

Met Thr Val Phe Phe Lys Thr Leu Arg Asn His Trp Lys Lys Thr Thr
5 10 15

Ala Gly Ile Cys Leu Leu Thr Trp Gly Gly His Trp Val Tyr Gly Lys
20 25 30

His Cys Asp Asn Leu Leu Arg Arg Ala Ala Cys Gln Glu Ala Gln Val
35 40 45

Phe Gly Asn Gln Leu Ile Pro Pro Asn Ala Gln Val Lys Lys Ala Thr
50 55 60

Val Phe Leu Asn Pro Ala Ala Cys Lys Gly Lys Ala Arg Thr Leu Phe
65 70 75 80

Glu Lys Asn Ala Ala Pro Ile Leu His Leu Ser Gly Met Asp Val Thr
85 90 95

Ile Val Lys Thr Asp Tyr Glu Gly Gln Ala Lys Lys Leu Leu Glu Leu
100 105 110

Met Glu Asn Thr Asp Val Ile Ile Val Ala Gly Gly Asp Gly Thr Leu
115 120 125

Gln Glu Val Ile Thr Gly Val Leu Arg Arg Glu Asp Glu Ala Thr Phe
130 135 140

Ser Lys Ile Pro Ile Gly Phe Ile Pro Leu Gly Gln Thr Ser Ser Leu
145 150 155 160

Ser Gln Thr Leu Phe Ala Glu Ser Gly Asn Lys Val Gln Arg Ile Thr
165 170 175

Asp Ala Thr Leu Ala Ile Val Lys Gly Glu Thr Val Pro Leu Asp Val

180 185 190

Leu Gln Ile Lys Gly Glu Lys Glu Gln Pro Val Phe Ala Leu Thr Gly

195 200 205

Leu Arg Trp Gly Ser Phe Arg Asp Ala Gly Val Ser Val Ser Arg Tyr

210 215 220

Trp Tyr Leu Gly Pro Leu Lys Thr Lys Ala Ala His Phe Phe Ser Thr

225 230 235 240

Leu Lys Glu Trp Pro Gln Thr His Gln Ala Ser Ile Ser Tyr Thr Gly

245 250 255

Pro Thr Glu Arg Pro Pro Ser Gly Ala Glu Glu Thr Pro Pro Arg Pro

260 265 270

Ser Leu Tyr Arg Arg Ile Leu Arg Arg Leu Ala Ser Tyr Trp Ala Gln

275 280 285

Pro Gln Gly Ala Leu Gln Glu Val Asn Pro Glu Val Trp Lys Glu Val

290 295 300

Gln Leu Ser Thr Leu Glu Leu Ser Ile Thr Thr Arg Asn Ser Gln Leu

305 310 315 320

Asp Leu Ala Ser Lys Glu Asp Phe Met Asn Ile Cys Met Glu Pro Asn

325 330 335

Thr Val Ser Lys Gly Asp Phe Ile Thr Ile Gly Ser Lys Lys Val Arg

340 345 350

Asp Pro Lys Leu His Val Glu Gly Thr Glu Cys Leu Gln Ala Ser Arg

355 360 365

Cys Thr Leu Leu Leu Pro Glu Gly Thr Gly Gly Ala Phe Ser Ile Asp

370 375 380

Ser Glu Glu Tyr Glu Ala Met Pro Val Glu Val Lys Leu Leu Pro Arg

385 390 395 400

Lys Leu Gln Phe Phe Cys Asp Pro Arg Lys Arg Glu Gln Leu Leu Gln

405 410 415

Ser Pro Ala Gln

420

<210> 4

<211> 1260

<212> DNA

<213> Bovine

<400> 4

ATGACTGTGT TCTTTAAAAC GCTTCGAAAT CATTGGAAGA AAACGACAGC TGGGATCTGC 60

CTGCTGACAT GGGGAGGCCA CTGGGTCTAT GGCAAACACT GTGATAACCT ACTAAGGAGA 120

GCAGCCTGTC AAGAAGCTCA GGTGTTTGGC AATCAACTTA TTCCTCCCAA TGCGCAAGTG 180

AAGAAGGCAA CTGTGTTTCT CAACCCTGCA GCTTGCAAAG GCAAAGCCAG GACTCTATTT 240

GAAAAAAATG CTGCCCCGAT TTTACACTTA TCTGGCATGG ATGTTACTAT CGTTAAGACA 300

GATTATGAGG GCCAGGCCAA GAAACTCCTG GAACTGATGG AAAACACAGA TGTGATCATT 360

GTTGCTGGAG GTGACGGGAC TTTGCAAGAG GTTATTACTG GAGTTCTTCG AAGAGAAGAT 420

GAGGCTACCT TCAGTAAGAT TCCCATCGGA TTCATCCCAC TGGGCCAGAC CAGTAGTTTG 480

AGTCAGACAC TCTTTGCCGA AAGTGGAAAC AAAGTCCAAC GTATTACAGA TGCTACACTT 540

GCCATTGTGA AAGGAGAGAC GGTTCCACTT GATGTCTTAC AGATAAAGGG TGAAAAGGAA 600

CAGCCTGTGT TTGCACTCAC CGGCCTTCGA TGGGGATCCT TCCGAGATGC TGGCGTCTCA 660

GTTAGCAGGT ACTGGTATCT TGGGCCTCTA AAAACCAAAG CAGCCCACTT TTTCAGCACT 720

CTTAAGGAGT GGCCTCAGAC TCATCAAGCC TCGATCTCGT ACACGGGACC TACAGAGAGA 780

CCTCCCAGTG GAGCAGAAGA GACCCCGCCC CGGCCCTCCT TGTATAGGAG AATATTACGG 840

AGGCTCGCGT CCTACTGGGC ACAACCGCAG GGAGCCCTGC AAGAGGTGAA CCCAGAGGTC 900

TGGAAGGAAG TGCAGTTGTC CACCCTTGAA CTGTCCATCA CAACCCGGAA CAGTCAGCTT 960

GACCTGGCAA GCAAAGAAGA CTTTATGAAC ATCTGCATGG AACCCAACAC CGTCAGCAAA 1020
GGAGACTTCA TCACTATAGG AAGTAAAAAG GTGAGAGATC CCAAGCTGCA TGTTGAGGGC 1080
ACCGAGTGTC TCCAAGCCAG CCGCTGCACT CTGCTGCTTC CAGAGGGCAC AGGGGGCGCC 1140
TTCAGCATCG ACAGTGAGGA GTACGAAGCG ATGCCCGTGG AGGTGAAGCT GCTCCCCCGG 1200
AAACTGCAGT TCTTCTGTGA CCCTCGCAAG AGGGAGCAGC TGCTGCAGAG CCCGGCCCAG 1260

<210> 5

<211> 422

<212> PRT

<213> Human

<400> 5

Met Thr Val Phe Phe Lys Thr Leu Arg Asn His Trp Lys Lys Thr Thr
5 10 15

Ala Gly Leu Cys Leu Leu Thr Trp Gly Gly His Trp Leu Tyr Gly Lys
20 25 30

His Cys Asp Asn Leu Leu Arg Arg Ala Ala Cys Gln Glu Ala Gln Val
35 40 45

Phe Gly Asn Gln Leu Ile Pro Pro Asn Ala Gln Val Lys Lys Ala Thr
50 55 60

Val Phe Leu Asn Pro Ala Ala Cys Lys Gly Lys Ala Arg Thr Leu Phe
65 70 75 80

Glu Lys Asn Ala Ala Pro Ile Leu His Leu Ser Gly Met Asp Val Thr
85 90 95

Ile Val Lys Thr Asp Tyr Glu Gly Gln Ala Lys Lys Leu Leu Glu Leu
100 105 110

Met Glu Asn Thr Asp Val Ile Ile Val Ala Gly Gly Asp Gly Thr Leu
115 120 125

Gln Glu Val Val Thr Gly Val Leu Arg Arg Thr Asp Glu Ala Thr Phe
130 135 140
Ser Lys Ile Pro Ile Gly Phe Ile Pro Leu Gly Glu Thr Ser Ser Leu
145 150 155 160
Ser His Thr Leu Phe Ala Glu Ser Gly Asn Lys Val Gln His Ile Thr
165 170 175
Asp Ala Thr Leu Ala Ile Val Lys Gly Glu Thr Val Pro Leu Asp Val
180 185 190
Leu Gln Ile Lys Gly Glu Lys Glu Gln Pro Val Phe Ala Met Thr Gly
195 200 205
Leu Arg Trp Gly Ser Phe Arg Asp Ala Gly Val Lys Val Ser Lys Tyr
210 215 220
Trp Tyr Leu Gly Pro Leu Lys Ile Lys Ala Ala His Phe Phe Ser Thr
225 230 235 240
Leu Lys Glu Trp Pro Gln Thr His Gln Ala Ser Ile Ser Tyr Thr Gly
245 250 255
Pro Thr Glu Arg Pro Pro Asn Glu Pro Glu Glu Thr Pro Val Gln Arg
260 265 270
Pro Ser Leu Tyr Arg Arg Ile Leu Arg Arg Leu Ala Ser Tyr Trp Ala
275 280 285
Gln Pro Gln Asp Ala Leu Ser Gln Glu Val Ser Pro Glu Val Trp Lys
290 295 300
Asp Val Gln Leu Ser Thr Ile Glu Leu Ser Ile Thr Thr Arg Asn Asn
305 310 315 320
Gln Leu Asp Pro Thr Ser Lys Glu Asp Phe Leu Asn Ile Cys Ile Glu
325 330 335

Pro Asp Thr Ile Ser Lys Gly Asp Phe Ile Thr Ile Gly Ser Arg Lys
340 345 350

Val Arg Asn Pro Lys Leu His Val Glu Gly Thr Glu Cys Leu Gln Ala
355 360 365

Ser Gln Cys Thr Leu Leu Ile Pro Glu Gly Ala Gly Gly Ser Phe Ser
370 375 380

Ile Asp Ser Glu Glu Tyr Glu Ala Met Pro Val Glu Val Lys Leu Leu
385 390 395 400

Pro Arg Lys Leu Gln Phe Phe Cys Asp Pro Arg Lys Arg Glu Gln Met
405 410 415

Leu Thr Ser Pro Thr Gln
420 422

<210> 6

<211> 1266

<212> DNA

<213> Human

<400> 6

ATGACGGTGT TCTTTAAAAC GCTTCGAAAT CACTGGAAGA AAACTACAGC TGGGCTCTGC 60

CTGCTGACCT GGGGAGGCCA TTGGCTCTAT GGAAAACACT GTGATAACCT CCTAAGGAGA 120

GCAGCCTGTC AAGAAGCTCA GGTGTTTGGC AATCAACTCA TTCCTCCCAA TGCACAAGTG 180

AAGAAGGCCA CTGTTTTTCT CAATCCTGCA GCTTGCAAAG GAAAAGCCAG GACTCTATTT 240

GAAAAAAATG CTGCCCCGAT TTTACATTTA TCTGGCATGG ATGTGACTAT TGTTAAGACA 300

GATTATGAGG GACAAGCCAA GAAACTCCTG GAACTGATGG AAAACACGGA TGTGATCATT 360

GTTGCAGGAG GAGATGGGAC ACTGCAGGAG GTTGTTACTG GTGTTCTTCG ACGAACAGAT 420

GAGGCTACCT TCAGTAAGAT TCCCATTGGA TTTATCCCAC TGGGAGAGAC CAGTAGTTTG 480

AGTCATACCC TCTTTGCCGA AAGTGGAAAC AAAGTCCAAC ATATTACTGA TGCCACACTT 540

GCCATTGTGA AAGGAGAGAC AGTTCCACTT GATGTCTTGC AGATCAAGGG TGAAAAGGAA 600

CAGCCTGTAT TTGCAATGAC CGGCCTTCGA TGGGGATCTT TCAGAGATGC TGGCGTCAAA 660

GTTAGCAAGT ACTGGTATCT TGGGCCTCTA AAAATCAAAG CAGCCCACTT TTTCAGCACT 720

CTTAAGGAGT GGCCTCAGAC TCATCAAGCC TCTATCTCAT ACACGGGACC TACAGAGAGA 780

CCTCCCAATG AACCAGAGGA GACCCCTGTA CAAAGGCCTT CTTTGTACAG GAGAATATTA 840

CGAAGGCTTG CGTCCTACTG GGCACAACCA CAGGATGCCC TTTCCCAAGA GGTGAGCCCG 900

GAGGTCTGGA AAGATGTGCA GCTGTCCACC ATTGAACTGT CCATCACAAC ACGGAATAAT 960

CAGCTTGACC CGACAAGCAA AGAAGATTTT CTGAATATCT GCATTGAACC TGACACCATC 1020

AGCAAAGGAG ACTTTATAAC TATAGGAAGT CGAAAGGTGA GAAACCCCAA GCTGCACGTG 1080

GAGGGCACGG AGTGTCTCCA AGCCAGCCAG TGCACTTTGC TTATCCCGGA GGGAGCAGGG 1140

GGCTCTTTTA GCATTGACAG TGAGGAGTAT GAAGCGATGC CTGTGGAGGT GAAACTGCTC 1200

CCCAGGAAGC TGCAGTTCTT CTGTGATCCT AGGAAGAGAG AACAGATGCT CACAAGCCCC 1260

ACCCAG 1266

<210> 7

<211> 421

<212> PRT

<213> Rat

<400> 7

Met Thr Thr Phe Phe Lys Thr Leu Arg Asn His Trp Lys Lys Thr Thr
5 10 15

Ala Gly Leu Cys Leu Leu Thr Trp Gly Gly His Trp Leu Tyr Gly Lys
20 25 30

His Cys Asp Asn Leu Leu Arg Arg Ala Ala Cys Gln Glu Ala Gln Val
35 40 45

Phe Gly Asn Gln Leu Ile Pro Pro Asn Ala Gln Val Lys Lys Ala Thr
50 55 60

Val Phe Leu Asn Pro Ala Ala Cys Lys Gly Lys Ala Arg Thr Leu Phe
65 70 75 80
Glu Lys Asn Ala Ala Pro Ile Leu His Leu Ser Gly Met Asp Val Thr
85 90 95
Val Val Lys Thr Asp Tyr Glu Gly Gln Ala Lys Lys Leu Leu Glu Leu
100 105 110
Met Glu Thr Thr Asp Val Ile Ile Val Ala Gly Gly Asp Gly Thr Leu
115 120 125
Gln Glu Val Val Thr Gly Val Leu Arg Arg Thr Asp Glu Ala Thr Phe
130 135 140
Ser Lys Ile Pro Ile Gly Phe Ile Pro Leu Gly Gln Thr Ser Ser Leu
145 150 155 160
Ser His Thr Leu Phe Ala Glu Ser Gly Asn Lys Val Gln His Val Thr
165 170 175
Asp Ala Ala Leu Ala Ile Val Lys Gly Glu Thr Val Pro Leu Asp Val
180 185 190
Leu Gln Ile Lys Gly Glu Lys Glu Gln Pro Val Tyr Ala Met Thr Gly
195 200 205
Leu Arg Trp Gly Ser Phe Arg Asp Ala Gly Val Lys Val Ser Lys Tyr
210 215 220
Trp Tyr Leu Gly Pro Leu Lys Thr Lys Ala Ala His Phe Phe Ser Thr
225 230 235 240
Leu Gln Glu Trp Pro Gln Thr His Gln Ala Ser Ile Ser Tyr Thr Gly
245 250 255
Pro Thr Glu Arg Pro Pro Ile Gly Pro Glu Asp Ala Ala Pro Arg Pro
260 265 270

Ser Leu Tyr Arg Arg Ile Leu Arg Arg Leu Ala Ser Phe Trp Ala Gln
275 280 285

Pro Gln Asp Ala Phe Ser Pro Glu Val Ser Pro Glu Val Trp Lys Asp
290 295 300

Val Gln Leu Ser Thr Ile Glu Leu Ser Ile Thr Thr Arg Asn Thr Gln
305 310 315 320

Leu Asp Leu Thr Ser Lys Glu Asp Phe Met Asn Ile Cys Ile Glu Pro
325 330 335

Asp Thr Val Ser Lys Gly Asp Phe Ile Ile Ile Gly Ser Lys Lys Val
340 345 350

Arg Asp Pro Gly Leu Arg Ala Ala Gly Thr Glu Cys Leu His Ala Ser
355 360 365

Arg Cys Thr Leu Ser Leu Pro Glu Gly Thr Glu Gly Ser Phe Ser Ile
370 375 380

Asp Ser Glu Glu Tyr Glu Ala Met Pro Val Glu Val Lys Leu Leu Pro
385 390 395 400

Arg Lys Leu Gln Phe Phe Cys Asp Pro Arg Lys Arg Glu Gln Met Leu
405 410 415

Gln Ser Thr Ser Gln
420 421

<210> 8

<211> 1263

<212> DNA

<213> Rat

<400> 8

ATGACCACAT TCTTTAAAAC ACTTCGAAAT CATTGGAAGA AAACTACAGC TGGCCTCTGC 60

TTGCTGACAT GGGGAGGTCA CTGGCTCTAT GGAAAGCACT GTGATAACCT GCTAAGAAGA 120
GCAGCTTGTC AAGAAGCTCA GGTGTTTGGC AACCAGCTCA TTCCTCCCAA TGCACAAGTG 180
AAGAAAGCCA CCGTCTTTCT CAATCCTGCA GCTTGCAAAG GCAAAGCCAG AACTCTATTT 240
GAAAAAAATG CTGCCCCAAT TTTACACCTG TCTGGCATGG ATGTGACTGT CGTCAAGACA 300
GATTATGAGG GACAAGCCAA GAAGCTCTTG GAGTTGATGG AAACGACCGA TGTGATCATT 360
GTTGCTGGAG GAGATGGCAC CCTGCAGGAG GTTGTTACTG GAGTACTTAG AAGAACAGAT 420
GAGGCTACCT TCAGTAAGAT TCCAATTGGA TTTATCCCGC TGGGACAGAC CAGTAGTTTG 480
AGTCACACCC TCTTTGCTGA AAGTGGAAAC AAAGTCCAAC ATGTCACAGA TGCCGCACTG 540
GCCATTGTGA AAGGGGAGAC TGTTCCACTT GACGTCTTGC AGATCAAGGG TGAAAAAGAA 600
CAGCCTGTCT ATGCCATGAC CGGCCTCCGG TGGGGATCCT TCAGAGATGC TGGCGTCAAA 660
GTTAGCAAGT ACTGGTATCT TGGTCCTCTA AAAACCAAAG CAGCCCACTT TTTCAGCACT 720
CTTCAGGAGT GGCCTCAGAC CCATCAGGCC TCCATCTCTT ACACGGGCCC TACAGAGAGA 780
CCTCCCATTG GGCCTGAAGA TGCTGCTCCC CGGCCTTCTC TGTACAGGAG AATATTACGT 840
AGGCTTGCCT CATTCTGGGC ACAGCCACAG GATGCCTTCT CCCCAGAGGT GAGCCCTGAG 900
GTCTGGAAAG ATGTACAACT GTCCACCATT GAACTGTCCA TCACAACCCG AAACACCCAA 960
CTTGACCTGA CGAGCAAAGA GGACTTTATG AACATTTGCA TTGAGCCTGA CACTGTCAGC 1020
AAAGGAGATT TCATAATCAT AGGCAGTAAG AAGGTGAGAG ACCCTGGTCT GCGAGCAGCC 1080
GGCACCGAGT GTCTCCACGC CAGCCGCTGC ACTCTGTCTC TTCCTGAGGG AACTGAGGGC 1140
TCCTTCAGCA TCGACAGCGA GGAGTATGAA GCCATGCCTG TGGAGGTCAA ACTCCTGCCC 1200
AGAAAACTTC AGTTCTTCTG TGATCCAAGG AAGAGAGAGC AAATGCTGCA GAGCACATCC 1260
CAG 1263

<210> 9

<211> 51

<212> DNA

<213> Human

<400> 9

ACGGTGTTCT TTAAAACGCT TCGAAATCAC TGGAAGAAAA CTACAGCTGG G 51

<210> 10

<211> 17

<212> PRT

<213> Rat

<400> 10

Thr Thr Phe Phe Lys Thr Leu Arg Asn His Trp Lys Lys Thr Thr Ala

1 5 10 15

Gly

17

<210> 11

<211> 51

<212> DNA

<213> Rat

<400> 11

ACCACATTCT TTAAAACACT TCGAAATCAT TGGAAGAAAA CTACAGCTGG C 51

<210> 12

<211> 30

<212> DNA

<213> Artificial Sequence

<220>

<223>

<400> 12

CAGCCGTGCA AATCCCTAAG AGAAGATGAC 30

<210> 13

<211> 26

<212> DNA

<213> Artificial Sequence

<220>

<223>

<400> 13

GCGGGGGACA GAGGAGGCCT TGGTTC 26

<210> 14

<211> 23

<212> DNA

<213> Artificial Sequence

<220>

<223>

<400> 14

TGGACCAGCC GTGCAAATCT CTA 23

<210> 15

<211> 24

<212> DNA

<213> Artificial Sequence

<220>

<223>

<400> 15

CCGGTGGCCT AAAGTGTGGT CTCA 24

<210> 16

<211> 22

<212> DNA

<213> Artificial Sequence

<220>

<223>

<400> 16

AGCGGCGGGC AGCGGAGAAG AT 22

<210> 17

<211> 22

<212> DNA

<213> Artificial Sequence

<220>

<223>

<400> 17

TGACACTGGG CCAGAAGCAC AC 22

<210> 18

<211> 366

<212> PRT

<213> Human

<400> 18

Met Trp Asn Ala Thr Pro Ser Glu Glu Pro Gly Phe Asn Leu Thr Leu
1 5 10 15

Ala Asp Leu Asp Trp Asp Ala Ser Pro Gly Asn Asp Ser Leu Gly Asp
20 25 30

Glu Leu Leu Gln Leu Phe Pro Ala Pro Leu Leu Ala Gly Val Thr Ala
35 40 45

Thr Cys Val Ala Leu Phe Val Val Gly Ile Ala Gly Asn Leu Leu Thr
50 55 60

Met Leu Val Val Ser Arg Phe Arg Glu Leu Arg Thr Thr Thr Asn Leu

65 70 75 80

Tyr Leu Ser Ser Met Ala Phe Ser Asp Leu Leu Ile Phe Leu Cys Met

85 90 95

Pro Leu Asp Leu Val Arg Leu Trp Gln Tyr Arg Pro Trp Asn Phe Gly

100 105 110

Asp Leu Leu Cys Lys Leu Phe Gln Phe Val Ser Glu Ser Cys Thr Tyr

115 120 125

Ala Thr Val Leu Thr Ile Thr Ala Leu Ser Val Glu Arg Tyr Phe Ala

130 135 140

Ile Cys Phe Pro Leu Arg Ala Lys Val Val Val Thr Lys Gly Arg Val

145 150 155 160

Lys Leu Val Ile Phe Val Ile Trp Ala Val Ala Phe Cys Ser Ala Gly

165 170 175

Pro Ile Phe Val Leu Val Gly Val Glu His Glu Asn Gly Thr Asp Pro

180 185 190

Trp Asp Thr Asn Glu Cys Arg Pro Thr Glu Phe Ala Val Arg Ser Gly

195 200 205

Leu Leu Thr Val Met Val Trp Val Ser Ser Ile Phe Phe Phe Leu Pro

210 215 220

Val Phe Cys Leu Thr Val Leu Tyr Ser Leu Ile Gly Arg Lys Leu Trp

225 230 235 240

Arg Arg Arg Arg Gly Asp Ala Val Val Gly Ala Ser Leu Arg Asp Gln

245 250 255

Asn His Lys Gln Thr Val Lys Met Leu Ala Val Val Val Phe Ala Phe

260 265 270

Ile Leu Cys Trp Leu Pro Phe His Val Gly Arg Tyr Leu Phe Ser Lys

275 280 285

Ser Phe Glu Pro Gly Ser Leu Glu Ile Ala Gln Ile Ser Gln Tyr Cys

290 295 300

Asn Leu Val Ser Phe Val Leu Phe Tyr Leu Ser Ala Ala Ile Asn Pro

305 310 315 320

Ile Leu Tyr Asn Ile Met Ser Lys Lys Tyr Arg Val Ala Val Phe Arg

325 330 335

Leu Leu Gly Phe Glu Pro Phe Ser Gln Arg Lys Leu Ser Thr Leu Lys

340 345 350

Asp Glu Ser Ser Arg Ala Trp Thr Glu Ser Ser Ile Asn Thr

355 360 365 366

<210> 19

<211> 366

<212> PRT

<213> Pig

<400> 19

Met Trp Asn Ala Thr Pro Ser Glu Glu Pro Gly Pro Asn Leu Thr Leu

1 5 10 15

Pro Asp Leu Gly Trp Asp Ala Pro Pro Glu Asn Asp Ser Leu Val Glu

20 25 30

Glu Leu Leu Pro Leu Phe Pro Thr Pro Leu Leu Ala Gly Val Thr Ala

35 40 45

Thr Cys Val Ala Leu Phe Val Val Gly Ile Ala Gly Asn Leu Leu Thr

50 55 60

Met Leu Val Val Ser Arg Phe Arg Glu Met Arg Thr Thr Thr Asn Leu

65 70 75 80

Tyr Leu Ser Ser Met Ala Phe Ser Asp Leu Leu Ile Phe Leu Cys Met
85 90 95
Pro Leu Asp Leu Phe Arg Leu Trp Gln Tyr Arg Pro Trp Asn Leu Gly
100 105 110
Asn Leu Leu Cys Lys Leu Phe Gln Phe Val Ser Glu Ser Cys Thr Tyr
115 120 125
Ala Thr Val Leu Thr Ile Thr Ala Leu Ser Val Glu Arg Tyr Phe Ala
130 135 140
Ile Cys Phe Pro Leu Arg Ala Lys Val Val Val Thr Lys Gly Arg Val
145 150 155 160
Lys Leu Val Ile Leu Val Ile Trp Ala Val Ala Phe Cys Ser Ala Gly
165 170 175
Pro Ile Phe Val Leu Val Gly Val Glu His Asp Asn Gly Thr Asp Pro
180 185 190
Arg Asp Thr Asn Glu Cys Arg Ala Thr Glu Phe Ala Val Arg Ser Gly
195 200 205
Leu Leu Thr Val Met Val Trp Val Ser Ser Val Phe Phe Phe Leu Pro
210 215 220
Val Phe Cys Leu Thr Val Leu Tyr Ser Leu Ile Gly Arg Lys Leu Trp
225 230 235 240
Arg Arg Lys Arg Gly Glu Ala Ala Val Gly Ser Ser Leu Arg Asp Gln
245 250 255
Asn His Lys Gln Thr Val Lys Met Leu Ala Val Val Val Phe Ala Phe
260 265 270
Ile Leu Cys Trp Leu Pro Phe His Val Gly Arg Tyr Leu Phe Ser Lys
275 280 285

Ser Leu Glu Pro Gly Ser Val Glu Ile Ala Gln Ile Ser Gln Tyr Cys
290 295 300
Asn Leu Val Ser Phe Val Leu Phe Tyr Leu Ser Ala Ala Ile Asn Pro
305 310 315 320
Ile Leu Tyr Asn Ile Met Ser Lys Lys Tyr Arg Val Ala Val Phe Lys
325 330 335
Leu Leu Gly Phe Glu Pro Phe Ser Gln Arg Lys Leu Ser Thr Leu Lys
340 345 350
Asp Glu Ser Ser Arg Ala Trp Thr Glu Ser Ser Ile Asn Thr
355 360 365 366

<210> 20
<211> 289
<212> PRT
<213> Human
<400> 20

Met Trp Asn Ala Thr Pro Ser Glu Glu Pro Gly Phe Asn Leu Thr Leu
1 5 10 15
Ala Asp Leu Asp Trp Asp Ala Ser Pro Gly Asn Asp Ser Leu Gly Asp
20 25 30
Glu Leu Leu Gln Leu Phe Pro Ala Pro Leu Leu Ala Gly Val Thr Ala
35 40 45
Thr Cys Val Ala Leu Phe Val Val Gly Ile Ala Gly Asn Leu Leu Thr
50 55 60
Met Leu Val Val Ser Arg Phe Arg Glu Leu Arg Thr Thr Thr Asn Leu
65 70 75 80
Tyr Leu Ser Ser Met Ala Phe Ser Asp Leu Leu Ile Phe Leu Cys Met

85 90 95

Pro Leu Asp Leu Val Arg Leu Trp Gln Tyr Arg Pro Trp Asn Phe Gly

100 105 110

Asp Leu Leu Cys Lys Leu Phe Gln Phe Val Ser Glu Ser Cys Thr Tyr

115 120 125

Ala Thr Val Leu Thr Ile Thr Ala Leu Ser Val Glu Arg Tyr Phe Ala

130 135 140

Ile Cys Phe Pro Leu Arg Ala Lys Val Val Val Thr Lys Gly Arg Val

145 150 155 160

Lys Leu Val Ile Phe Val Ile Trp Ala Val Ala Phe Cys Ser Ala Gly

165 170 175

Pro Ile Phe Val Leu Val Gly Val Glu His Glu Asn Gly Thr Asp Pro

180 185 190

Trp Asp Thr Asn Glu Cys Arg Pro Thr Glu Phe Ala Val Arg Ser Gly

195 200 205

Leu Leu Thr Val Met Val Trp Val Ser Ser Ile Phe Phe Phe Leu Pro

210 215 220

Val Phe Cys Leu Thr Val Leu Tyr Ser Leu Ile Gly Arg Lys Leu Trp

225 230 235 240

Arg Arg Arg Arg Gly Asp Ala Val Val Gly Ala Ser Leu Arg Asp Gln

245 250 255

Asn His Lys Gln Thr Val Lys Met Leu Gly Gly Ser Gln Arg Ala Leu

260 265 270

Arg Leu Ser Leu Ala Gly Pro Ile Leu Ser Leu Cys Leu Leu Pro Ser

275 280 285

Leu

289

<210> 21

<211> 289

<212> PRT

<213> Pig

<400> 21

Met Trp Asn Ala Thr Pro Ser Glu Glu Pro Gly Pro Asn Leu Thr Leu
1 5 10 15

Pro Asp Leu Gly Trp Asp Ala Pro Pro Glu Asn Asp Ser Leu Val Glu
20 25 30

Glu Leu Leu Pro Leu Phe Pro Thr Pro Leu Leu Ala Gly Val Thr Ala
35 40 45

Thr Cys Val Ala Leu Phe Val Val Gly Ile Ala Gly Asn Leu Leu Thr
50 55 60

Met Leu Val Val Ser Arg Phe Arg Glu Met Arg Thr Thr Thr Asn Leu
65 70 75 80

Tyr Leu Ser Ser Met Ala Phe Ser Asp Leu Leu Ile Phe Leu Cys Met
85 90 95

Pro Leu Asp Leu Phe Arg Leu Trp Gln Tyr Arg Pro Trp Asn Leu Gly
100 105 110

Asn Leu Leu Cys Lys Leu Phe Gln Phe Val Ser Glu Ser Cys Thr Tyr
115 120 125

Ala Thr Val Leu Thr Ile Thr Ala Leu Ser Val Glu Arg Tyr Phe Ala
130 135 140

Ile Cys Phe Pro Leu Arg Ala Lys Val Val Val Thr Lys Gly Arg Val
145 150 155 160

Lys Leu Val Ile Leu Val Ile Trp Ala Val Ala Phe Cys Ser Ala Gly
165 170 175

Pro Ile Phe Val Leu Val Gly Val Glu His Asp Asn Gly Thr Asp Pro
180 185 190

Arg Asp Thr Asn Glu Cys Arg Ala Thr Glu Phe Ala Val Arg Ser Gly
195 200 205

Leu Leu Thr Val Met Val Trp Val Ser Ser Val Phe Phe Phe Leu Pro
210 215 220

Val Phe Cys Leu Thr Val Leu Tyr Ser Leu Ile Gly Arg Lys Leu Trp
225 230 235 240

Arg Arg Lys Arg Gly Glu Ala Ala Val Gly Ser Ser Leu Arg Asp Gln
245 250 255

Asn His Lys Gln Thr Val Lys Met Leu Gly Gly Ser Gln Cys Ala Leu
260 265 270

Glu Leu Ser Leu Pro Gly Pro Leu His Ser Ser Cys Leu Phe Ser Ser
275 280 285

Pro
289

<210> 22

<211> 34

<212> DNA

<213> Artificial Sequence

<220>

<223>

<400> 22

GTCGACCATG TGGAACGCGA CCCCCAGCGA GGAG 34

<210> 23

<211> 31

<212> DNA

<213> Artificial Sequence

<220>

<223>

<400> 23

GCTAGCGGAG AGATGGGATG TGCTGTCATG T 31

<210> 24

<211> 364

<212> PRT

<213> Rat

<400> 24

Met Trp Asn Ala Thr Pro Ser Glu Glu Pro Glu Pro Asn Val Thr Leu
1 5 10 15

Asp Leu Asp Trp Asp Ala Ser Pro Gly Asn Asp Ser Leu Pro Asp Glu
20 25 30

Leu Leu Pro Leu Phe Pro Ala Pro Leu Leu Ala Gly Val Thr Ala Thr
35 40 45

Cys Val Ala Leu Phe Val Val Gly Ile Ser Gly Asn Leu Leu Thr Met
50 55 60

Leu Val Val Ser Arg Phe Arg Glu Leu Arg Thr Thr Thr Asn Leu Tyr
65 70 75 80

Leu Ser Ser Met Ala Phe Ser Asp Leu Leu Ile Phe Leu Cys Met Pro
85 90 95

Leu Asp Leu Val Arg Leu Trp Gln Tyr Arg Pro Trp Asn Phe Gly Asp

100 105 110

Leu Leu Cys Lys Leu Phe Gln Phe Val Ser Glu Ser Cys Thr Tyr Ala

115 120 125

Thr Val Leu Thr Ile Thr Ala Leu Ser Val Glu Arg Tyr Phe Ala Ile

130 135 140

Cys Phe Pro Leu Arg Ala Lys Val Val Val Thr Lys Gly Arg Val Lys

145 150 155 160

Leu Val Ile Leu Val Ile Trp Ala Val Ala Phe Cys Ser Ala Gly Pro

165 170 175

Ile Phe Val Leu Val Gly Val Glu His Glu Asn Gly Thr Asp Pro Arg

180 185 190

Asp Thr Asn Glu Cys Arg Ala Thr Glu Phe Ala Val Arg Ser Gly Leu

195 200 205

Leu Thr Val Met Val Trp Val Ser Ser Val Phe Phe Phe Leu Pro Val

210 215 220

Phe Cys Leu Thr Val Leu Tyr Ser Leu Ile Gly Arg Lys Leu Trp Arg

225 230 235 240

Arg Arg Gly Asp Ala Ala Val Gly Ala Ser Leu Arg Asp Gln Asn His

245 250 255

Lys Gln Thr Val Lys Met Leu Ala Val Val Val Phe Ala Phe Ile Leu

260 265 270

Cys Trp Leu Pro Phe His Val Gly Arg Tyr Leu Phe Ser Lys Ser Phe

275 280 285

Glu Pro Gly Ser Leu Glu Ile Ala Gln Ile Ser Gln Tyr Cys Asn Leu

290 295 300

Val Ser Phe Val Leu Phe Tyr Leu Ser Ala Ala Ile Asn Pro Ile Leu

305 310 315 320

Tyr Asn Ile Met Ser Lys Lys Tyr Arg Val Ala Val Phe Lys Leu Leu

325 330 335

Gly Phe Glu Ser Phe Ser Gln Arg Lys Leu Ser Thr Leu Lys Asp Glu

340 345 350

Ser Ser Arg Ala Trp Thr Lys Ser Ser Ile Asn Thr

355 360 364

<210> 25

<211> 1092

<212> DNA

<213> Rat

<400> 25

ATGTGGAACG CGACCCCCAG CGAGGAGCCG GAGCCTAACG TCACGTTGGA CCTGGATTGG 60

GACGCTTCCC CCGGCAACGA CTCACTGCCT GACGAACTGC TGCCGCTGTT CCCCGCTCCG 120

CTGCTGGCAG GCGTCACCGC CACCTGCGTG GCGCTCTTCG TGGTGGGCAT CTCAGGCAAC 180

CTGCTCACTA TGCTGGTGGT GTCCCGCTTC CGCGAGCTGC GCACCACCAC CAACCTCTAC 240

CTGTCCAGCA TGGCCTTCTC GGATCTGCTC ATCTTCCTGT GCATGCCGCT GGACCTCGTC 300

CGCCTCTGGC AGTACCGGCC CTGGAACTTC GGCGACCTGC TCTGCAAACT CTTCCAGTTT 360

GTCAGCGAGA GCTGCACCTA CGCCACGGTC CTCACCATCA CCGCGCTGAG CGTCGAGCGC 420

TACTTCGCCA TCTGCTTCCC TCTGCGGGCC AAGGTGGTGG TCACTAAGGG CCGCGTGAAG 480

CTGGTCATCC TTGTCATCTG GGCCGTGGCT TTCTGCAGCG CGGGGCCCAT CTTCGTGCTG 540

GTGGGCGTGG AGCACGAAAA CGGCACAGAT CCCCGGGACA CCAACGAATG CCGCGCCACC 600

GAGTTCGCTG TGCGCTCTGG GCTGCTCACC GTCATGGTGT GGGTGTCCAG CGTCTTCTTC 660

TTTCTACCGG TCTTCTGCCT CACTGTGCTC TACAGTCTCA TCGGGAGGAA GCTATGGCGG 720

AGACGCGGAG ATGCAGCGGT GGGCGCCTCG CTCCGGGACC AGAACCACAA GCAGACAGTG 780

AAGATGCTTG CTGTGGTGGT GTTTGCTTTC ATCCTCTGCT GGCTGCCCTT CCACGTGGGA 840

```
AGATACCTCT TTTCCAAGTC CTTCGAGCCT GGCTCTCTGG AGATCGCTCA GATCAGCCAG    900
TACTGCAACC TGGTGTCCTT TGTCCTCTTC TACCTCAGCG CTGCCATCAA CCCCATTCTG    960
TACAACATCA TGTCCAAGAA GTACCGGGTG GCAGTGTTCA AACTGCTAGG ATTTGAATCC   1020
TTCTCCCAGA GAAAGCTTTC CACTCTGAAG GATGAGAGTT CCCGGGCCTG GACAAAGTCG   1080
AGCATCAACA CA                                                       1092
```

## Description

FIELD OF THE INVENTION

The present invention relates to a novel ligand polypeptide to a growth hormone secretagogue receptor (GHSR), which is an orphan G protein-coupled receptor protein, and a DNA encoding the same, etc.

BACKGROUND ART

A variety of hormones and neurotransmitters control or regulate the functions of the body via specific receptors present on cell membranes. Many of these receptors are coupled to guanine nucleotide-binding proteins (hereinafter, sometimes referred to as G proteins) to mediate the intracellular signal transduction via activation of the G proteins. These receptors possess a common structural framework comprising seven transmembrane domains and are thus referred to collectively as G protein-coupled receptor proteins or seven transmembrane receptors.

These hormones and neurotransmitters interact with G protein-coupled receptor proteins and, via the interactive reaction, regulate important functions for a living body such as maintenance of homeostasis, reproduction, development of individuals, metabolism, growth, control of the nervous, circulatory, immune, digestive or metabolic system, sensory adaptation, or the like. As such it is known that there are receptor proteins to various hormones or neurotransmitters for regulating the functions of the body and these receptor proteins play key roles for regulating these functions. However, it often remains unclear if there are any other unknown substances (hormones, neurotransmitters, etc.) and receptors to these substances.

In recent years, a polymerase chain reaction (hereinafter abbreviated as PCR) has been performed to explore a DNA encoding a novel receptor protein, utilizing the nature of a G protein-coupled receptor protein to show similarity in part of the amino acid sequence in its structure, and many ligand-unknown, so-called orphan G protein-coupled receptor proteins have been cloned (Libert, F., et al., Science, 244, 569-572, 1989, Welch, S.K., et al., Biochem. Biophys. Res. Commun., 209, 606-613, 1995; Marchese, A., et al., Genomics, 23, 609-618, 1994; Marchese, A., Genomics, 29, 335-344, 1995). Also, novel G protein-coupled receptor proteins have been found one after another by random sequencing of genomic DNA or cDNA (Nomura, N., et al., DNA Research, 1, 27-35, 1994). Only one general method for determining ligands to these G protein-coupled receptor proteins was to deduce ligands from similarity in primary structure of G protein-coupled receptor proteins. However, many orphan G protein-coupled receptor proteins have low homology to known receptors. Practically, it was difficult to deduce ligands only from similarity in primary structure, except that they are receptor subtypes of known ligands. On the other hand, since many orphan G protein-coupled receptor proteins were found by gene analysis, it is anticipated that there would be many other unknown ligands corresponding to those proteins. However, only a few ligands to G protein-coupled receptor proteins have actually been identified.

So far, several GHS (growth hormone secretagogues) including GHRP-6 (growth hormone-releasing peptide-6) have been developed as artificial peptides having a potent growth hormone secretion promoting activity. It has been found that these substances have a GH secretion promoting activity both in vitro and in vivo, and are engaged with a mechanism different from that of an endogenous peptide GHRH (growth hormone-releasing hormone) identified in the past. Moreover, GHS such as GHRP-6 or the like synergistically promotes GH secretion when administered simultaneously in combination with GHRH, and is thus regarded as useful in terms of clinical application (Hormone-to-Rinsho, 47 (5), 33-43 (1999)). Growth hormone secretagogue receptor (GHSR), on which GHS such as GHRP-6 or the like acts, was cloned by Howard, et al. in 1996 (Science, 273, 974-977 (1996)) and found to be a G protein-coupled receptor. However, the endogenous ligand to GHSR has not been identified so far. The mechanism of controlling GH secretion remains unclear in many points, and it has been waited to identify endogenous ligands to GHSR.

Endogenous ligands to GHSR, which is an orphan G protein-coupled receptor expressed in the organ such as central nervous system, circulatory system, genital system, immune system, digestive organs and the like, are considered to be useful as drugs and for screening a compound capable of changing the binding property to GHSR but the structure and function are unknown yet.

DISCLOSURE OF THE INVENTION

The present inventors have succeeded in isolating and identifying a polypeptide recognized by the receptor protein as a ligand under the index of a specific cell stimulating (signal transduction) activity measured, etc., using a cell in which a GHSR-encoding cDNA has been expressed by an appropriate means.

The inventors have further discovered that a compound that can alter the binding property between the activator ligand and a GHSR described above can be screened.

Thus, the present invention relates to the following features:

(1) A polypeptide containing the same or substantially the same amino acid sequence as the amino acid sequence represented by SEQ ID NO: 1, its amide or ester, or a salt thereof.

(2) The polypeptide, its amide or ester, or a salt thereof, described in (1) above, wherein substantially the same amino acid sequence is the amino acid sequence represented by SEQ ID NO: 10.

(3) The polypeptide, its amide or ester, or a salt thereof, described in (1) above, which contains the same or substantially the same amino acid sequence represented by SEQ ID NO: 3, SEQ ID NO: 5 or SEQ ID NO: 7.

(4) A DNA containing a DNA encoding the polypeptide described in (1) above.

(5) The DNA described in (4) above, which contains the base sequence represented by SEQ ID NO: 2, SEQ ID NO: 4, SEQ ID NO: 6, SEQ ID NO: 8, SEQ ID NO: 9 or SEQ ID NO: 11.

(6) An antibody to the polypeptide, its amide or ester, or a salt thereof, described in (1) above.

(7) A diagnostic agent comprising the DNA described in (5) above or the antibody described in (6) above.

(8) An antisense DNA having a complementary or substantially complementary base sequence to the DNA described in (5) above and capable of suppressing expression of said DNA.

(9) An agent comprising the polypeptide or its amide or ester, or a salt thereof, described in (1) above.

(10) A pharmaceutical comprising the polypeptide or its amide or ester, or a salt thereof, described in (1) above.

(11) The pharmaceutical described in (10) above, which is a prophylactic/therapeutic agent for diseases associated with deficiency of growth hormone.

(12) The pharmaceutical described in (10) above, which is a prophylactic/therapeutic agent for pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome.

(13) A method for screening a compound or its salt that promotes or inhibits the activity of the polypeptide, its amide or ester, or a salt thereof, described in (1) above, which comprises using the polypeptide, its amide or ester, or a salt thereof described in (1) above.

(14) The method for screening described in (13) above, which comprises using the polypeptide, its amide or ester, or a salt thereof, described in (1) above, and a polypeptide containing the same or substantially the same amino acid sequence represented by SEQ ID NO: 18, its amide or ester, or a salt thereof, or a partial peptide thereof, its amide or ester, or a salt thereof.

(15) A kit for screening a compound or its salt that promotes or inhibits the activity of the polypeptide, its amide or ester, or a salt thereof, described in (1) above, comprising the polypeptide, its amide or ester, or a salt thereof, described in (1) above, and a polypeptide containing the same or substantially the same amino acid sequence represented by SEQ ID NO: 18, its amide or ester, or a salt thereof, or a partial peptide thereof, its amide or ester, or a salt thereof.

(16) A compound or its salt that promotes or inhibits the activity of the polypeptide, its amide or ester, or a salt thereof, described in (1) above, obtainable using the screening method described in (13) above or the screening kit described in (15) above.

(17) A growth hormone secretion modulator comprising the compound or its salt described in (16) above.

(18) A prophylactic/therapeutic agent for ① pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, or ② acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, comprising the compound or its salt described in (16) above.

(19) Use of the polypeptide, its amide or ester, or a salt thereof, described in (1) above, for the preparation of a pharmaceutical for the prevention/treatment of pituitary dwarfism, Turner's syndrome, chronic renal failure, chondrodystrophia, adult pituitary deficiency, Down's syndrome, Silver's 5 syndrome, osteogenesis imperfecta or juvenile chronic arthrosis syndrome.

(20) A method for preventing/treating pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, which comprises administering the polypeptide, its amide or ester, or a salt thereof, described in (1) above to a mammal.

BRIEF DESCRIPTION OF THE DRAWINGS

FIG. 1 shows a pattern of the bovine hypothalamus-derived active fraction in RESOURCE S detected in EXAMPLE 3, which was separated using Sephasil C8 SC 2.1/10 mins., and the results of the activity detected to be specific to CHO-GHSR-23.

FIG. 2 shows the amino acid sequence corresponding to bovine gene sequence obtained in EXAMPLE 5 (continued to FIG. 3).

FIG. 3 shows the amino acid sequence corresponding to bovine gene sequence obtained in EXAMPLE 5 (continued from FIG. 2).

FIG. 4 shows the amino acid sequence corresponding to human gene sequence obtained in EXAMPLE 6 (continued to FIG. 5).

FIG. 5 shows the amino acid sequence corresponding to human gene sequence obtained in EXAMPLE 6 (continued from FIG. 4).

FIG. 6 shows comparison (alignment) among the amino acid sequence corresponding to bovine gene sequence obtained in EXAMPLE 5, the amino acid sequence corresponding to human gene sequence obtained in EXAMPLE 6 and the amino acid sequence corresponding to rat gene sequence obtained in EXAMPLE 7.

FIG. 7 shows the amino acid sequence corresponding to rat gene sequence obtained in EXAMPLE 7 (continued to FIG. 8) .

FIG. 8 shows the amino acid sequence corresponding to rat gene sequence obtained in EXAMPLE 7 (continued from FIG. 7).

BEST MODE FOR CARRYING OUT THE INVENTION

Specifically with respect to GHSR to the polypeptide, its amide or ester, or a salt thereof, in accordance with the present invention, not only known GHSR or its salts, etc. described above but also the following features can be given.

(21) A GHSR containing the same or substantially the same amino acid sequence as the amino acid sequence shown by SEQ ID NO: 18, or a salt thereof.

(22) The GHSR or a salt thereof described in (21) above, wherein substantially the same amino acid sequence is the amino acid sequence shown by SEQ ID NO: 19, SEQ ID NO: 20, SEQ ID NO: 21 or SEQ ID NO: 24.

(23) The GHSR or a salt thereof described in (21) above, which is a protein containing an amino acid sequence, wherein at least 1 to 30, preferably 1 to 10, all inclusive, amino acids are deleted in the amino acid sequence shown by SEQ ID NO: 18; an amino acid sequence, wherein at least 1 to 30, preferably 1 to 10, all inclusive, amino acids are added to (or inserted into) the amino acid sequence shown by SEQ ID NO: 18; or an amino acid sequence, wherein at least 1 to 30, preferably 1 to 10, all inclusive, amino acids are replaced with other amino acids in the amino acid sequence shown by SEQ ID NO: 18.

As the partial peptide of the GHSR (hereinafter sometimes merely referred to as the partial peptide), any partial peptide can be used so long as it is a partial peptide of the GHSR described above. For example, a part of the GHSR protein molecule which is exposed to the outside of a cell membrane can be used as long as it has a ligand binding activity.

Specifically, the partial peptide of the polypeptide having the amino acid sequence represented by SEQ ID NO: 18, SEQ ID NO: 19, SEQ ID NO: 20, SEQ ID NO: 21 or SEQ ID NO: 24 is a peptide containing the parts which have been analyzed to be extracellular domains (hydrophilic domains) of GHSR. A peptide containing a hydrophobic domain part can be used as well. In addition, the peptide that contains each domain separately may be employed, and peptides containing plural domains together may be employed as well.

The number of the amino acids in the partial peptide of GHSR may be at least 20 or more, preferably at least 50 or more, and more preferably at least 100 or more, in the amino acid sequence which constitutes the GHSR described above.

The substantially the same amino acid sequence includes an amino acid sequence having at least about 70% homology, preferably at least about 80% homology, more preferably at least about 90% homology, and most preferably at least about 95% homology, to these amino acid sequences.

Herein the term "substantially equivalent activities" refers to the same significance as defined hereinafter. Examples of the salts of GHSR and its partial peptides include salts of the polypeptide of the present invention, which will be later described.

Throughout the specification, the term "substantially the same" is used to mean that the activities of a polypeptide or protein, e.g., the binding activity between a ligand and a receptor (GHSR), physiological properties, etc. are substantially the same. Substitution, deletion, addition or insertion of an amino acid does not often cause any significant alteration in physiological properties or chemical properties of the polypeptide or protein; in this case, such a polypeptide that undergoes substitution, deletion, addition or insertion is considered to be substantially the same as the polypeptide that does not undergo substitution, deletion, addition or insertion. Substantially the same substituent of an amino acid in the amino acid sequence can be selected from, e.g., other amino acids of the class to which the amino acid belongs. Examples of non-polar (hydrophobic) amino acids include alanine, leucine, isoleucine, valine, proline, phenylalanine, tryptophan, methionine and the like. Examples of polar (neutral) amino acids are glycine, serine, threonine, cysteine, tyrosine, asparagine, glutamine, and the like. Examples of positively charged (basic) amino acids are arginine, lysine, histidine, and the like. Examples of negatively charged (acidic) amino acids include aspartic acid, glutamic acid, and the like.

The polypeptide, its amide or ester, or a salt thereof, according to the present invention is a ligand to GHSR and refers specifically to the polypeptide containing the same or substantially the same amino acid sequence represented by SEQ ID NO: 1, its amide or ester, or a salt thereof, etc.

The polypeptide of the present invention, its amide or ester, or a salt thereof (hereinafter sometimes merely referred to as the polypeptide of the present invention), a method for manufacturing the same and use thereof are described below in more detail.

The polypeptide of the present invention may be any polypeptide so long as it is derived from any tissues (e. g., pituitary gland, pancreas, brain, kidney, liver, genital gland, thyroid gland, gall bladder, spinal cord, adrenal, skin, muscle, lung, digestive tract, blood vessel, heart, etc.), cells or the like of human and other warm-blooded animals (e. g., guinea pig, rat, mouse, swine, sheep, bovine, monkey, etc.) and contains the same or substantially the same amino acid sequence as the amino acid sequence shown by SEQ ID NO: 1. Examples of the polypeptide of the present invention include, in addition to the polypeptides containing the amino acid sequence shown by SEQ ID NO: 1, a polypeptide which has substantially equivalent activity to the polypeptide containing the amino acid sequence shown by SEQ ID NO: 1 (e.g., a polypeptide containing the amino acid sequence shown by SEQ ID NO: 3, SEQ ID NO: 5, SEQ ID NO: 7 or SEQ ID NO: 10, etc.) and the like. Examples of the substantially equivalent activity include a receptor (ligand) binding activity, a signal transduction activity, GH secretion promoting activity (action) and the like. The term substantially equivalent is used to mean that the nature of the receptor (ligand) binding activity, etc. is equivalent. Therefore, differences in degree such as a level of the receptor (ligand) binding activity and quantitative factors such as a molecular weight of the polypeptide may be present and allowable.

Specific examples of the polypeptides which contain substantially the same amino acid sequence as the amino acid sequence shown by SEQ ID NO: 1 are polypeptides containing the amino acid sequence shown by SEQ ID NO: 1 in which the second amino acid (Val) from the N-terminus is substituted with other amino acid (e.g., Ala, Leu, Ile, Thr, Pro, Phe, Trp, Met, Gly, Ser, Cys, Tyr, Asn, Gln, Arg, Lys, His, Asp, Glu). Of these polypeptides, preferred is a polypeptide containing the amino acid sequence shown by SEQ ID NO: 1 in which the second amino acid (Val) from the N-terminus is substituted with Thr (SEQ ID NO: 10), or the like.

Specific examples of the polypeptide which contains substantially the same amino acid sequence as that shown by SEQ ID NO: 1 include a polypeptide containing the amino acid sequence shown by SEQ ID NO: 3, SEQ ID NO: 5 or SEQ ID NO: 7, and the like. Throughout the present specification, the polypeptides are represented in accordance with the conventional way of describing peptides, that is, the N-terminus (amino terminus) at the left hand and the C-terminus (carboxyl terminus) at the right hand. In the polypeptides containing the amino acid sequences such as 1) the amino acid sequence shown by SEQ ID NO: 1, 2) the amino acid sequence shown by SEQ ID NO: 3, 3) the amino acid sequence shown by SEQ ID NO: 5, 4) the amino acid sequence shown by SEQ ID NO: 7, 5) the amino acid sequence shown by SEQ ID NO: 10, etc., the C-terminus is normally in the form of a carboxyl group (-COOH) or a carboxylate ($-COO^-$) but may also be in the form of an amide ($-CONH_2$) or an ester (-COOR). Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a phenyl-$C_{1-2}$ alkyl group such as benzyl, phenethyl, benzhydryl, etc.; a $C_{7-14}$ aralkyl such as an α-naphthyl-$C_{1-2}$ alkyl group, e.g., α-naphthylmethyl, etc.; and the like. In addition, pivaloyloxymethyl or the like which is used widely as an ester for oral administration may be used as well.

Furthermore, the polypeptides of the present invention include variants of the above polypeptides, wherein the amino group at the N-terminal methionine residue is protected with a protecting group (e.g., a $C_{1-6}$ acyl group such as a $C_{2-6}$ alkanoyl group, e.g., formyl group, acetyl group, etc.); those wherein the N-terminal side is cleaved *in vivo* and the glutamyl group thus formed is pyroglutaminated; those wherein a substituent (e.g., -OH, -SH, -COOH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins having sugar chains such as so-called glycoproteins, and the like.

As the salts of the polypeptide of the present invention, there may be used salts with physiologically acceptable bases (e.g., alkali metal salts, etc.) or acids (e.g., inorganic acids or organic acids). In particular, physiologically acceptable acid addition salts are preferable. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide of the present invention may be manufactured by a method used to purify a polypeptide from human or other warm-blooded animal tissues or cells. Alternatively, the polypeptide of the present invention may also be manufactured by a modification of the methods for synthesizing polypeptides, which will be described hereinafter.

Furthermore, the polypeptide of the present invention may also be manufactured by culturing a transformant containing a DNA encoding the polypeptide, as will also be later described.

Where the polypeptide is produced from human or warm-blooded animal tissues or cells, human or warm-blooded animal tissues or cells are homogenized, then extracted with an acid, an organic solvent or the like, and the extract is purified/isolated by means of salting-out, dialysis, gel filtration, or a combination of chromatography techniques such as reverse phase chromatography, ion exchange chromatography, affinity chromatography, and the like.

As stated above, the polypeptide of the present invention can be manufactured by publicly known methods for polypeptide synthesis or by cleaving a polypeptide containing the polypeptide of the present invention with an appropriate peptidase. For the peptide synthesis methods, for example, either solid phase synthesis method or liquid phase synthesis method may be used. That is, partial peptides or amino acids that may constitute the polypeptide of the present invention are condensed with the residual portion. When the product has protecting groups, the desired peptide can be obtained by removing the protecting groups. The publicly known condensation and protecting group removal methods include the methods described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the polypeptide of the present invention may be purified and isolated by a combination of conventional purification methods such as solvent extraction, distillation, column chromatography, liquid chromatography, recrystallization, and the like. When the polypeptide obtained by the above methods is in a free form, the polypeptide can be converted into an appropriate salt by a publicly known method; conversely when the polypeptide is obtained in a salt form, it can be converted into a free form by a publicly known method.

To synthesize the amides of the polypeptide, commercially available resins for peptide synthesis that are suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4,-dimethoxyphenyl-Fmoc-aminoethyl)phenoxy resin, etc. Using these resins, amino acids, in which $\alpha$-amino groups and functional groups on the side chains are appropriately protected, are condensed on the resin, in accordance with the order of the sequence of the objective peptide, by various condensation methods publicly known in the art. At the end of the reaction, the peptide is excised from the resin and at the same time, the protecting groups are removed. Then, intramolecular disulfide bond-forming reaction is performed in a highly diluted solution to obtain the desired polypeptide.

For condensation of the protected amino acids described above, a variety of activating reagents for peptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt, etc.) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be appropriately chosen from solvents that are known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and suitable mixtures of these solvents. The reaction temperature is appropriately chosen from the range known to be applicable to peptide binding reactions and is normally selected in the range of approximately -20°C to 50°C. The activated amino acid derivatives are used generally in an excess of 1.5 to 4 times. The condensation is tested using the ninhydrin reaction; when the test reveals that the condensation is insufficient, the condensation can be completed by repeating the condensation reaction without removal of the protecting groups. When the condensation is still insufficient even after repeating the reaction, unreacted amino acids are acetylated with acetic anhydride or acetylimidazole to cancel any possible adverse affect on the subsequent reaction.

Examples of the protecting groups used to protect the amino groups in the starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting group shown above by R for a carboxyl group include, in addition to those examples for the $C_{1-6}$ alkyl group, the $C_{3-8}$ cycloalkyl and the $C_{7-14}$ aralkyl described hereinabove, 2-adamantyl, 4-nitrobenzyl, 4-methoxybenzyl, 4-chlorobenzyl, phenacyl and benzyloxycarbonylhydrazide, t-butoxycarbonylhydrazide, tritylhydrazide, and the like.

The hydroxyl group in serine and threonine can be protected through, for example, their esterification or etherification. Examples of groups appropriately used for the esterification include a lower alkanoyl group such as acetyl, etc., an aroyl group such as benzoyl group, and a group derived from carbon such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting materials include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino groups in the starting material, the corresponding phosphoric amides are employed.

To remove (split off) the protecting groups, there are used catalytic hydrogenation under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a solution mixture of these acids; a treatment with a base such as diisopropylethylamine, triethylamine, piperidine or piperazine; and reduction with sodium in liquid ammonia. Removal of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethyl sulfide, 1,4-butanedithiol or 1,2-ethanedithiol. Furthermore, 2,4-dinitrophenyl group known as a protecting group for the imidazole of histidine is removed by a treatment with thiophenol. Formyl group used as a protecting group of the indole in tryptophan is removed by the aforesaid acid treatment in the presence of 1,2-ethanedithiol or 1,4-butanedithiol, as well as by a treatment with an alkali such as a dilute sodium hydroxide solution, dilute ammonia, etc.

Protection of functional groups that should not be involved in the reaction of the starting materials, protecting groups, removal of the protecting groups and activation of functional groups involved in the reaction may be appropriately selected from publicly known groups and publicly known means.

In another method for obtaining the amides of the polypeptide, for example, the α-carboxyl group of the carboxyl terminal amino acid is first amidated; the peptide chain is then extended from the amino group side to a desired length. Thereafter, a peptide in which only the protecting group of the N-terminal α-amino group in the peptide chain has been removed and a peptide in which only the protecting group of the C-terminal carboxyl group has been removed are produced. The two peptides are condensed in a mixture of the solvents described above. The details of the condensation reaction are the same as described above. After the protected peptide obtained by the condensation is purified, all the protecting groups are removed by the method described above to give the desired crude polypeptide. This crude polypeptide is purified by various known purification means. Lyophilization of the major fraction gives the amide of the desired polypeptide.

To prepare the esterified polypeptide, the α-carboxyl group of the carboxy terminal amino acid is condensed with a desired alcohol to prepare the amino acid ester, which is followed by procedures similar to the preparation of the amidated polypeptide above to give the ester of the desired polypeptide.

Any polypeptide is usable as the polypeptide of the present invention so long as it contains the same or substantially the same amino acid sequence as the amino acid sequence represented by SEQ ID NO: 1 described above and possesses the same activities as those of the polypeptide, e.g., the binding activity to GHSR, activity of signal transduction to GHSR, or GH secretion promoting activity (action). Examples of such a polypeptide include a peptide having the amino acid sequence shown by SEQ ID NO: 3, SEQ ID NO: 5, SEQ ID NO: 7 and SEQ ID NO: 10 described above, etc..

Moreover, the polypeptide of the present invention can be used as an antigen for preparing an antibody to the polypeptide. As such a polypeptide that can be used as the antigen, partial peptides such as N-terminal peptide, C-terminal peptide, a peptide at the central part, etc. of the polypeptide in the present invention may be employed as well, in addition to the polypeptides of the present invention described above.

The DNA encoding the polypeptide of the present invention may be any DNA so long as it contains a DNA encoding a polypeptide containing the same or substantially the same amino acid sequence represented by SEQ ID NO: 1. Also, the DNA may be any one of genomic DNA, genomic DNA library, cDNA derived from the tissues or cells described above, cDNA library derived from the tissues or cells described above and synthetic DNA. The vector used for the library may be any of bacteriophage, plasmid, cosmid, phagemid and the like. In addition, the DNA can be directly amplified by reverse transcriptase polymerase chain reaction (hereinafter abbreviated as RT-PCR technique) using an RNA fraction prepared from the tissues or cells described above.

Herein, the polypeptide containing the same or substantially the same amino acid sequence as the amino acid sequence shown by SEQ ID NO: 1 includes the amino acid sequence shown by SEQ ID NO: 3, SEQ ID NO: 5, SEQ ID NO: 7, or SEQ ID NO: 10, as described above. The DNA containing the polypeptide-encoding DNA containing the amino acid sequence shown by SEQ ID NO: 1 is, for example, a DNA containing a DNA having the base sequence shown by SEQ ID NO: 2, a DNA containing a DNA having the base sequence shown by SEQ ID NO: 9, and the like; the DNA containing the polypeptide-encoding DNA containing the amino acid sequence shown by SEQ ID NO: 3 is, e.g., a DNA containing a DNA having the base sequence shown by SEQ ID NO: 4, etc.; the DNA containing the polypeptide-encoding DNA containing the amino acid sequence shown by SEQ ID NO: 5 is, e.g., a DNA containing a DNA having the base sequence shown by SEQ ID NO: 6, etc.; the DNA containing the polypeptide-encoding DNA containing the amino acid sequence shown by SEQ ID NO: 7 is, e.g., a DNA containing a DNA having the base sequence shown by SEQ ID NO: 8, etc.; and the DNA containing the polypeptide-encoding DNA containing the amino acid sequence shown by SEQ ID NO: 10 is, e.g., a DNA containing a DNA having the base sequence shown by SEQ ID NO: 11, etc.

Examples of the DNA containing a DNA encoding the polypeptide having the same or substantially the same amino acid sequence as the amino acid sequence shown by SEQ ID NO: 1 include a DNA containing a DNA having a base sequence with homology of at least about 80%, preferably at least about 90%, more preferably at least about 95%, and most preferably at least about 98%, to the base sequence shown by SEQ ID NO: 2 or SEQ ID NO: 9.

Examples of the DNA containing a DNA encoding the polypeptide having the same or substantially the same amino acid sequence as the amino acid sequence shown by SEQ ID NO: 1 include DNAs containing 1) the base sequence represented by SEQ ID NO: 2 or SEQ ID NO: 9, of which at least 1 or 2 (preferably approximately 1 to 10, and more preferably 1 or 2) bases are deleted; 2) the base sequence represented by SEQ ID NO: 2 or SEQ ID NO: 9, of which at least 1 or 2 (preferably approximately 1 to 10, and more preferably 1 or 2) bases are added; 3) the base sequence represented by SEQ ID NO: 2 or SEQ ID NO: 9, of which at least 1 or 2 (preferably approximately 1 to 10, and more preferably 1 or 2) bases are inserted; 4) in the amino acid sequence represented by SEQ ID NO: 2 or SEQ ID NO: 9, of which at least 1 or 2 (preferably approximately 1 to 10, and more preferably 1 or 2) bases are substituted by other bases; or 5) a combination of the above base sequences. More specifically, there are employed (1) a mammal-derived DNA that is hybridizable under stringent conditions to the sequence possessed by a DNA containing a DNA encoding the polypeptide of the present invention containing the same or substantially the same amino acid sequence as the amino acid sequence represented by SEQ ID NO: 1; (2) a DNA that is not hybridizable due to degeneracy of genetic code to the sequence possessed by the DNA containing a DNA encoding the polypeptide of the present invention containing the same or substantially the same amino acid sequence as the amino acid sequence represented by SEQ ID NO: 1 and to the sequence defined in (1) but encoding a polypeptide having the same amino acid sequence, and the like. The hybridization can be carried out by publicly known methods or by a modification of known methods. The stringent conditions described above are, for example, conditions of 42°C, 50% formamide, 4 x SSPE (1 x SSPE = 150 mM NaCl, 10 mM $NaH_2PO_4 \cdot H_2O$, 1 mM EDTA, pH 7.4), 5 x Denhardt's solution and 0.1% SDS.

As the DNA hybridizable to the sequence possessed by a DNA containing a DNA encoding the polypeptide of the present invention containing the same or substantially the same amino acid sequence as the amino acid sequence represented by SEQ ID NO: 1, there are employed, e.g., a DNA containing a base sequence having homology of at least about 70%, preferably at least about 80%, more preferably at least about 90%, and most preferably at least about 95%, to the base sequence shown by SEQ ID NO: 2 or SEQ ID NO: 9, and the like.

DNA fragments containing a partial base sequence of the DNA encoding the polypeptide containing 1) the amino acid sequence shown by SEQ ID NO: 1, 2) the amino acid sequence shown by SEQ ID NO: 3, 3) the amino acid sequence shown by SEQ ID NO: 5, 4) the amino acid sequence shown by SEQ ID NO: 7, 5) the amino acid sequence shown by SEQ ID NO: 10, etc. are also preferably used as probes for DNA detection.

The DNA encoding the polypeptide of the present invention may also be manufactured by the following genetic engineering techniques.

For cloning of the DNA that fully encodes the polypeptide of the present invention, the target DNA may be either amplified from the DNA library, etc. *supra* by publicly known PCR using synthetic DNA primers containing a partial base sequence of the polypeptide of the present invention, or the DNA inserted into an appropriate vector can be selected by hybridization with a labeled DNA fragment or synthetic DNA that encodes a part or entire region of the polypeptide of the present invention. The hybridization can be carried out by the methods described, for example, in Molecular Cloning (2nd ed.; J. Sambrook et al., Cold Spring Harbor Lab. Press, 1989), etc. When a commercially available library is used, the hybridization may also be performed in accordance with the protocol described in the instructions attached.

The cloned DNA encoding the polypeptide of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the polypeptide of the present invention can be manufactured, for example, by (a) excising the desired DNA fragment from the DNA encoding the polypeptide of the present invention, (b) and then ligating the DNA fragment to an appropriate expression vector downstream of a promoter in the vector.

Examples of the vector include plasmids derived form *E. coli* (e.g., pBR322, pBR325, pUC12, pUC13). plasmids derived from *Bacillus subtilis* (e.g., pUB110, pTP5, pC194), plasmids derived from yeast (e.g., pSH19, pSH15), bacteriophages such as λ phage, etc., animal viruses such as retrovirus, vaccinia virus, baculovirus, etc.

The promoter used in the present invention may be any promoter if it matches well with a host to be used for gene expression.

When an animal cell is used as a host for transformation, there may be utilized SV40-derived promoter, retrovirus promoter, metallothionein promoter, heat shock promoter, cytomegalovirus promoter, SRα promoter, etc. Where the host is bacteria of the genus Escherichia, preferred examples include trp promoter, T7 promoter, lac promoter, recA promoter, λPL promoter, lpp promoter, etc. When bacteria of the genus Bacillus are used as the host, preferred examples are SPO1 promoter, SPO2 promoter, penP promoter, etc. When yeast is used as the host, preferred examples are PHO5 promoter, PGK promoter, GAP promoter, ADH1 promoter, GAL promoter, etc. When insect cells are used as the host, preferred examples include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^-$, G418 resistance), etc. In particular, when DHFR gene is used as the selection marker using CHO ($dhfr^-$) cells, selection may also be made on thymidine free media.

If necessary, a signal sequence that matches with a host is added to the N-terminal side of the polypeptide or its partial peptide. Examples of the signal sequence that can be utilized are phoA signal sequence, OmpA signal sequence, etc. when bacteria of the genus Escherichia are used as the host; α-amylase signal sequence, subtilisin signal sequence, etc. when bacteria of the genus Bacillus are used as the host; mating factor α(MFα) signal sequence, invertase signal sequence, etc. when yeast is used as the host; and insulin signal sequence, α-interferon signal sequence, antibody molecule signal sequence, etc. when animal cells are used as the host, respectively.

Using the vector comprising the DNA encoding the polypeptide thus constructed, transformants can be prepared.

Examples of the host, which may be employed, are bacteria belonging to the genus Escherichia, bacteria belonging to the genus Bacillus, yeast, insects or insect cells and animal cells, etc.

Specific examples of the bacteria belonging to the genus Escherichia include *Escherichia coli* K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, $AH22R^-$, NA87-11A, DKD-5D, 20B-12, etc.

As the insect, for example, a larva of Bombyx mori can be used [Maeda et al., Nature, 315, 592 (1985)].

Examples of insect cells include, for the virus AcNPV, *Spodoptera frugiperda* cell (Sf cell), MG1 cell derived from mid-intestine of *Trichoplusia ni*, High Five™ cell derived from egg of *Trichoplusia ni,* cells derived from *Mamestra brassicae*, cells derived from *Estigmena acrea,* etc.; and for the virus BmNPV, *Bombyx mori* N cell (BmN cell), etc. is used. Examples of the Sf cell which can be used are Sf9 cell (ATCC CRL1711) and Sf21 cell [both cells are described in Vaughn, J. L. et al., In vitro, 13, 213-217 (1977)], etc.

Examples of animal cells include monkey cell COS-7, Vero cell, Chinese hamster cell CHO, DHFR gene deficient Chinese hamster cell CHO ($dhfr^-$ CHO cell), mouse L cell, mouse 3T3 cell, mouse myeloma cell, human HEK293 cell, human FL cell, 293 cell, C127 cell, BALB3T3 cell, Sp-2/O cell, etc.

Bacteria belonging to the genus Escherichia can be transformed, for example, by the method described in Proc. Natl. Acad. Sci. U.S.A., 69, 2110 (1972) or Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Proc. Natl. Acad. Sci. U.S.A., 75, 1929 (1978).

Insect cells or insects can be transformed, for example, according to the method described in Bio/Technology, 6, 47-55(1988), etc.

Animal cells can be transformed, for example, according to the method described in Virology, 52, 456 (1973).
Methods for introducing the expression vectors into the cells include, for example, the lipofection method [Felgner, P. L., et al., Proceedings of the National Academy of Sciences of the United States of America, 84, 7413 (1987)], the calcium phosphate method [Graham, F. L. and van der Eb, A., J. Virology, 52, 456-467 (1973)], the electroporation method [Nuemann, E. et al., EMBO J., 1, 841-845 (1982)], etc.
As described above, transformants transformed by the expression vector containing the DNA encoding the polypeptide of the present invention are obtained.
For stably expressing the polypeptide of the present invention using animal cells, there is applicable a method of selecting the cells by clone selection in which the aforesaid expression vectors transfected to animal cells are introduced into chromosomes. Specifically, transformants are selected using as an index the selection marker described above. Further by repeated clone selections on the transformants thus obtained using the selection marker, stable animal cell line capable of highly expressing the polypeptide of the present invention can be obtained. Furthermore, when the dhfr gene is used as the selection marker, cultivation can be performed by gradually increasing a level of MTX, resistant cells are selected thereby to amplify the DNA encoding the polypeptide of the present invention or its partial peptide in the cells together with the dhfr gene. Thus, the animal cell line of higher expression can be obtained.
The transformant described above is cultivated under conditions that the DNA encoding the polypeptide of the present invention can express, thereby to produce and accumulate the polypeptide of the present invention. Thus, the polypeptide of the present invention can be produced.
Where the host is bacteria belonging to the genus Escherichia or the genus Bacillus, the transformant can be appropriately cultivated in a liquid medium that contains materials required for growth of the transformant, such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrates, corn steep liquor, peptone, casein, meat extract, soybean cake, potato extract, etc. Examples of the inorganics are calcium chloride, sodium dihydrogenphosphate, magnesium chloride, etc. In addition, yeast, vitamins, growth promoting factors, etc. may also be added to the medium. Preferably, pH of the medium is adjusted to about 5 to about 8.
A preferred example of the medium for cultivation of the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid may be added to the medium thereby to activate the promoter efficiently.
Where the bacteria belonging to the genus Escherichia are used as the host, the transformant is usually cultivated at about 15 to about 43°C for about 3 hours to about 24 hours. If necessary, the culture may be aerated or agitated.
Where the bacteria belonging to the genus Bacillus are used as the host, the transformant is cultivated generally at about 30°C to about 40°C for about 6 hours to about 24 hours. If necessary, the culture can be aerated or agitated.
Where yeast is used as the host, the transformant is cultivated, for example, in Burkholder's minimal medium [Bostian, K. L. et al., Proc. Natl. Acad. Sci. U.S.A., 77, 4505 (1980)] or in SD medium [Bitter, G. A. et al., Proc. Natl. Acad. Sci. U.S.A., 81, 5330 (1984)] supplemented with 0.5% Casamino acid. Preferably, pH of the medium is adjusted to about 5 to about 8. In general, the transformant is cultivated at about 20°C to about 35°C for about 24 hours to about 72 hours. If necessary, the culture may be aerated or agitated.
Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture may be aerated or agitated.
Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture may be aerated or agitated.
Especially when the CHO ($dhfr^-$) cell and the dhfr gene are employed as the selection markers, it is preferred to use substantially thymidine-free DMEM supplemented with dialyzed bovine fetal serum.
The polypeptide of the present invention can be separated and purified from the culture described above by the following procedures.
When the polypeptide of the present invention is extracted from the culture or cells, after cultivation the transformant or cell is collected by a publicly known method and suspended in an appropriate buffer. The transformant or cell is then disrupted by publicly known methods such as ultrasonication, a treatment with lysozyme and/or freeze-thaw cycling, followed by centrifugation, filtration, etc. Thus, the crude extract of the polypeptide can be obtained. The buffer used for the procedures may contain a protein modifier such as urea or guanidine hydrochloride, or a surfactant such as Triton X-100 (trademark, hereinafter sometimes abbreviated as TM), etc.

When the polypeptide is secreted in the culture broth, after completion of the cultivation the supernatant can be separated from the transformant or cell to collect the supernatant by a publicly known method.

The polypeptide of the present invention contained in the thus-obtained culture supernatant or the extract can be purified by appropriately combining the publicly known methods for separation and purification. Such known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charges such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectric focusing, chromatofocusing, etc.; and the like.

When the polypeptide of the present invention thus obtained is in a free form, it can be converted into the salt by publicly known methods or modifications thereof. On the other hand, when the polypeptide is obtained in a salt form, it can be converted into the free form or a different salt form by publicly known methods or modifications thereof.

The polypeptide of the present invention produced by the recombinant can be treated, prior to or after the purification, with an appropriate protein modifying enzyme so that the polypeptide can be suitably modified or partially removed. Examples of the protein modifying enzyme include trypsin, chymotrypsin, arginyl endopeptidase, protein kinase, glycosidase and the like.

The presence of the thus formed polypeptide of the present invention can be determined by an enzyme immunoassay using a specific antibody, or the like.

The DNA encoding the polypeptide of the present invention or the polypeptide of the present invention can be used: 1) for the survey of physiological activities that the polypeptide of the present invention possesses, 2) for preparing synthetic oligonucleotide probes or primers for PCR, 3) for acquiring DNAs encoding ligands to precursor proteins of GHSR, 4) for the development of the receptor-binding assay system using the expression system of recombinant receptor protein and screening of a candidate drug, 5) for acquiring antibodies and antisera, 6) for the development of diagnostic agents using DNAs, RNAs, antibodies or antisera, 7) as agents for regulating GH (growth hormone)(prophylactic/therapeutic agents for diseases associated with deficiency of GH or diseases aassociated with over-secretion of GH), 8) for the development of (a) pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, or (b) acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, etc., 9) for gene therapy, and the like.

In particular, the receptor-binding assay system using the expression system of recombinant GHSR described hereinafter can be used for screening GHSR agonists or antagonists specific to a warm-blooded animal including human. These agonists or antagonists may be employed as agents for the prevention/treatment of various diseases.

Furthermore, with respect to 7) and 8) described above, the GHSR recognizes the polypeptide of the present invention as a ligand and therefore, the polypeptide of the present invention or the DNA encoding the same is useful as safe and low toxic drug. The polypeptide of the present invention or the DNA encoding the same is associated with the GH secretion promoting activity, etc., and can thus be used as the therapeutic/prophylactic agent for diseases such as pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, and the like.

When the polypeptide of the present invention or the DNA encoding the same is used as the drugs described above, its pharmaceutical preparations can be prepared in a conventional manner. The polypeptide or the DNA can be used orally, for example, in the form of tablets with sugar coating or enteric coating, if necessary, capsules, elixirs, microcapsules etc., or parenterally in the form of injectable preparations such as a sterile solution, a suspension, etc. in water or with other pharmaceutically acceptable liquid. These preparations can be manufactured by mixing the compound described above with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

Where the DNA of the present invention is used, the DNA alone may be administered, or may be inserted into an appropriate vector such as retrovirus vector, adenovirus vector, adenovirus-associated virus vector, etc. and then administered in a conventional manner.

Additives miscible with tablets, capsules etc. include a binder such as gelatin, corn starch, tragacanth, gum arabic, etc.; an excipient such as crystalline cellulose, etc.; a swelling agent such as corn starch, gelatin, alginic acid, etc.; a lubricant such as magnesium stearate; a sweetening agent such as sucrose, lactose, or saccharin; and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80(™) and HCO-50), etc. Examples of the oily medium include sesame oil, soybean oil, etc., which may also be used in combination with a dissolution aid such as benzyl benzoate, benzyl alcohol, etc.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, or the like. The thus-prepared liquid for injection is normally filled in an appropriate ampoule.

Since the thus obtained pharmaceutical preparation is safe and low toxic, the preparation can be administered to, e.g., human or other mammals (e.g., mouse, rat, guinea pig, rabbit, sheep, swine, bovine, cat, dog, monkey, etc.).

The dose of the polypeptide of the present invention or the DNA encoding the same varies depending on condition, etc. In oral administration, the dose is normally about 0.1 mg to about 100 mg, preferably about 1.0 to about 50 mg, and more preferably about 1.0 to about 20 mg per day for the adult patient with chronic renal failure (as 60 kg body weight). In parenteral administration, the single dose varies depending on subject to be administered, target organ, conditions, method for administration, etc. but it is advantageous to administer the active ingredient intravenously at a daily dose of about 0.01 to about 30 mg, preferably about 0.1 to about 20 mg, and more preferably about 0.1 to about 10 mg for the adult patient with chronic renal failure (as 60 kg body weight). For other animal species, the corresponding dose as converted per 60 kg body weight can be administered.

The polypeptide of the present invention, production thereof and applications thereof are described below in more detail.

Applications of the polypeptide of the present invention, the DNA encoding the polypeptide, and antibodies, etc. are specifically described below.

(1) prophylactic/therapeutic agent for polypeptide deficiency

The polypeptide of the present invention and the DNA encoding the polypeptide of the present invention may be employed also as a prophylactic/therapeutic agent for deficiency of the polypeptide or GHSR, depending upon the activities of the polypeptide of the present invention on the GHSR.

When a patient has a reduced level of the polypeptide of the present invention or GHSR in his or her body so that the patient cannot expect the ligand physiological activities (GH secretion promoting action, etc.), (a) the polypeptide of the present invention or the DNA encoding the polypeptide of the present invention is administered to the patient to express the same, or (b) the polypeptide of the present invention or the DNA encoding the polypeptide of the present invention is inserted into brain cells, etc. to express the same followed by transplanting the brain cells to the patient, whereby the polypeptide level is increased in the brain cells of the patient to sufficiently exhibit the activity of the polypeptide. Therefore, the polypeptide of the present invention or the DNA encoding the polypeptide of the present invention can be employed as a safe and low toxic prophylactic/therapeutic agent, etc., for deficiency of the polypeptide.

Where the DNA described above is used as the therapeutic agent described above, the DNA alone may be administered, or the DNA may be inserted into an appropriate vector such as retrovirus vector, adenovirus vector, adenovirus-associated virus vector, etc. and then administered, by the same means as in the case of using the DNA encoding the polypeptide of the present invention as drugs described above.

(2) Method for quantification of GHSR to the polypeptide

Since the polypeptide of the present invention is capable of binding to GHSR or its salts or the partial peptides of the receptor protein or salts thereof, the *in vivo* level of the GHSR or its salts or the partial peptides of the GHSR or salts thereof can be quantified in a high sensitivity.

This method for quantification can be used in combination with, e.g., the competitive method. That is, the level of GHSR or its salts, or the partial peptides of GHSR or its salts in a sample fluid can be measured by bringing the sample fluid in contact with the polypeptide of the present invention.

Specifically, the quantification can be performed by the following method (1) or (2) publicly known or its modifications:

(1) Hiroshi Irie (ed.): "Radioimmunoassay" (1974, published by Kodansha, Japan); and
(2) Hiroshi Irie (ed.): "Radioimmunoassay, Second Series" (1979, published by Kodansha, Japan).
(3) Method for screening the compound that alters the binding property between GHSR and the polypeptide of the present invention or its salts (the compound that promotes or inhibits the activity of the polypeptide of the present invention)

By using the GHSR or its salts or its partial peptides or salts thereof, or by constructing the expression system of recombinant GHSR and using the receptor-binding assay system using the expression system, compounds (e.g., peptides, proteins, non-peptide compounds, synthetic compounds, fermentation products, etc.) or salts thereof that alter the binding property between the polypeptide of the present invention and the GHSR (the compounds that promote or inhibit the activity of the polypeptide of the present invention) can be screened. Examples of such compounds include compounds showing GHSR-mediated cell-stimulating activities (e.g., the activities that promote release of arachidonic acid, release of acetylcholine, release of intracellular $Ca^{2+}$, intracellular cAMP production, intracellular cGMP production, inositol phosphate production, changes in cell membrane potential, phosphorylation of intracellular proteins, activation of c-fos, pH reduction, etc.)(namely, GHSR agonists) and compounds having no such cell-stimulating activities (namely, GHSR antagonists). The term "alter the binding property to the ligand" includes both the case in which the binding to the ligand is inhibited and the case in which the binding to the ligand is promoted.

That is, the present invention provides a method for screening the compound or its salts that alter the binding property between the polypeptide of the present invention and the GHSR described above (the compound that promotes or inhibits the activity of the polypeptide of the present invention), which comprises comparing (i) the case that the polypeptide of the present invention is brought into contact with the GHSR or its salts or the partial peptide of the GHSR or its salts, and (ii) the case that the polypeptide of the present invention and a test compound are brought into contact with the aforesaid GHSR or its salts or the partial peptide of the GHSR or its salts.

In the screening method of the present invention, the comparison is made by measuring, e.g., the amount of the ligand bound to the GHSR or its partial peptide, the cell-stimulating activity, etc., (i) when the polypeptide of the present invention is brought into contact with the aforesaid GHSR or the partial peptide of the GHSR, and (ii) when the polypeptide of the present invention and a test compound are brought into contact with the aforesaid GHSR or the partial peptide of the GHSR; and comparing (i) and (ii).

More specifically, the screening method of the present invention provides the following features.

1) A method for screening the compound or salts thereof that alter the binding property between the polypeptide of the present invention and GHSR (the compound that promotes or inhibits the activity of the polypeptide of the present invention), which comprises measuring the binding amounts of the labeled polypeptide of the present invention bound to the aforesaid GHSR or its salts or its partial peptide or its salts, when the labeled polypeptide of the present invention is brought in contact with the GHSR described above or its salts or the partial peptide of the GHSR or its salts and when the labeled polypeptide of the present invention and a test compound are brought in contact with the GHSR or its salts or the partial peptide of the GHSR or its salts and, comparing the binding amounts.
2) A method for screening the compound or salts thereof that alter the binding property between the polypeptide of the present invention and GHSR (the compound that promotes or inhibits the activity of the polypeptide of the present invention), which comprises measuring the binding amounts of the labeled polypeptide of the present invention bound to a GHSR-containing cell or membrane fraction of the cell, when the labeled polypeptide of the present invention is brought in contact with the GHSR-containing cell or membrane fraction of the cell and when the labeled polypeptide of the present invention and a test compound are brought in contact with the GHSR-containing cell or membrane fraction of the cell and, comparing the binding amounts.
3) A method for screening the compound or salts thereof that alter the binding property between the polypeptide of the present invention and GHSR (the compound that promotes or inhibits the activity of the polypeptide of the present invention), which comprises measuring the binding amounts of the labeled polypeptide of the present invention bound to the aforesaid GHSR, when the labeled polypeptide of the present invention is brought in contact with the GHSR expressed on a cell membrane by culturing a transformant containing the DNA encoding the GHSR, and when the labeled polypeptide of the present invention and a test compound are brought in contact with the GHSR expressed on a cell membrane by culturing a transformant containing the DNA encoding the GHSR and, comparing the binding amounts.
4) A method for screening the compound or salts thereof that alter the binding property between the polypeptide of the present invention and GHSR (the compound that promotes or inhibits the activity of the polypeptide of the present invention), which comprises measuring GHSR-mediated cell stimulating activities (e.g., activities that promote or inhibit release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.), when a compound for activating GHSR (e.g., the polypeptide of the present invention) is brought in contact with a cell containing GHSR and when a compound for activating GHSR and a test compound are brought in contact with the cell containing GHSR and, comparing the GHSR-mediated cell stimulating activities.

5) A method for screening the compound or salts thereof that alter the binding property between the polypeptide of the present invention and GHSR (the compound that promotes or inhibits the activity of the polypeptide of the present invention), which comprises measuring GHSR-mediated cell stimulating activities (e.g., activities that promote or inhibit release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.), when a compound for activating GHSR (e.g., the polypeptide of the present invention) is brought in contact with the GHSR expressed on a cell membrane by culturing a transformant containing the DNA encoding the GHSR and when a compound for activating the GHSR and a test compound are brought in contact with the GHSR expressed on a cell membrane by culturing a transformant containing the DNA encoding GHSR and, comparing the GHSR-mediated cell stimulating activities.

The screening methods of the present invention are specifically described below.

First, the GHSR used for the screening methods of the present invention may be any GHSR so long as it contains the aforesaid GHSR or the partial peptide of the GHSR. Preferred are membrane fractions from the organs of human or other warm-blooded animals. Since it is very difficult to obtain human-derived organs, the GHSR expressed in large quantities by use of recombinants is suitable as GHSR for the screening.

For producing GHSR, the method described in Science, 273, 974-977 (1966) or the methods for manufacturing the polypeptide of the present invention described above, etc. are used.

When the GHSR-containing cells or cell membrane fractions are employed in the screening methods of the present invention, the cells or cell membrane fractions may be prepared by the method which will be described hereinafter.

Where the GHSR-containing cells are used, the cells may be fixed using glutaraldehyde, formalin, etc. The fixation can be made by a publicly known method.

The cells containing GHSR refer to host cells that have expressed GHSR. Examples of the host cells include *Escherichia coli, Bacillus subtilis,* yeast, insect cells, animal cells, etc., described above. The GHSR-expressed host cells may be produced similarly by the aforesaid method for producing transformants transformed by the expression vector containing the polypeptide of the present invention.

The cell membrane fraction is a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 minute to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the expressed GHSR and membrane components such as cell-derived phospholipids and membrane proteins.

The amount of GHSR in the GHSR-containing cells or in the membrane fractions is preferably $10^3$ to $10^8$ molecules per cell, more preferably $10^5$ to $10^7$ molecules per cell. As the amount of expression increases, the ligand binding activity per unit of membrane fraction (specific activity) is promoted so that not only the highly sensitive screening system can be constructed but also large quantities of samples can be assayed with the same lot.

To perform the methods 1) through 3) for screening the compound that alters the binding property between the polypeptide of the present invention and GHSR (the compound that promotes or inhibits the activity of the polypeptide of the present invention), an appropriate GHSR fraction and the labeled polypeptide of the present invention are employed. The GHSR fraction is preferably a naturally occurring GHSR fraction or a recombinant GHSR fraction having an activity equivalent to that of the natural GHSR fraction. Herein, the equivalent activity is intended to mean a ligand binding activity, or the like, equivalent to that of the natural GHSR fraction. As the labeled ligand, there are used a labeled ligand, a labeled ligand analog compound, etc. For example, a ligand which is labeled with [3H], [$^{125}$I], [$^{14}$C], [$^{35}$S], etc. can be utilized.

Specifically, the compound that alters the binding property between the polypeptide of the present invention and the GHSR is screened by the following procedures. First, a standard receptor preparation is prepared by suspending GHSR-containing cells or membrane fractions thereof in a buffer suitable for the screening. Any buffer can be used so long as it does not interfere with ligand-receptor binding, such buffers including a phosphate buffer or a Tris-HCl buffer, having pH of 4 to 10 (desirably pH of 6 to 8). For the purpose of minimizing non-specific binding, a surfactant such as CHAPS, Tween-80™ (Kao-Atlas Inc.), digitonin, deoxycholate, etc. may optionally be added to the buffer. Further for the purpose of suppressing the degradation of the receptor or the polypeptide of the present invention by a protease, a protease inhibitor such as PMSF, leupeptin, E-64 (manufactured by Peptide Institute, Inc.) and pepstatin may also be added to the buffer. A given amount (5,000 cpm to 500,000 cpm) of the labeled polypeptide of the present invention is added to 0.01 ml to 10 ml of the receptor solution and at the same time, a test compound of $10^{-10}$ to $10^{-7}$ M is allowed to be co-present. To determine the amount of non-specific binding (NSB), a reaction tube added with unlabeled polypeptide of the present invention in a large excess is also provided. The reaction is carried out at 0°C to 50°C, preferably 4 to 37°C for 20 minutes to 24 hours, preferably 30 minutes to 3 hours. After completion of the reaction, the reaction mixture is filtrated through a glass fiber filter paper, etc. and washed with a suitable volume of the same buffer. The residual radioactivity in the glass fiber filter paper is then measured by means of a liquid scintillation counter or a γ-counter. When the count ($B_0$-NSB) obtained by subtracting non-specific binding count (NSB) from the count ($B_0$) in the absence of an antagonistic substance is taken as 100%, the test compound giving a non-specific binding (B-NSB) of, e.g., 50% or less can be selected as a candidate substance having a competitive inhibitory activity.

The method 4) or 5) described above for screening the compound that alters the binding property between the polypeptide of the present invention and GHSR (the compound that promotes or inhibits the activity of the polypeptide of the present invention) can be performed as follows. The GHSR-mediated cell-stimulating activities (e.g., the activities that promote or inhibit release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.) may be assayed by a publicly known method, or by using an assay kit commercially available. Specifically, the GHSR-containing cells are first cultured on a multiwell plate, etc. Prior to the screening, the medium is previously replaced with fresh medium or with an appropriate non-cytotoxic buffer, followed by incubation for a given period of time in the presence of a test compound, etc. Subsequently, the cells are extracted or the supernatant is recovered and the resulting product is quantified by the respective procedures. Where it is difficult to detect the production of the index substance (e.g., arachidonic acid, etc.) for the cell-stimulating activity due to degrading enzymes contained in the cells, an inhibitor against such a degradation enzyme may be added prior to the assay. For detecting activities such as the cAMP production suppression activity, the baseline production in the cells is promoted by forskolin or the like. Then, the suppressing effect on the promoted baseline production can be detected.

For screening by measurement of the cell-stimulating activity, a suitable cell in which GHSR has been expressed is required. Such a cell that the GHSR of the present invention has been expressed is desirably the aforesaid recombinant GHSR-expressed cell line, and the like.

Examples of the test compound include a peptide, a protein, a non-peptide compound, a synthetic compound, a fermentation product, a cell extract, a plant extract, an animal tissue extract and the like.

The kit for screening the compound or its salts that alter the binding property between the polypeptide of the present invention or its precursor protein and GHSR (the compound that promotes or inhibits the activity of the polypeptide of the present invention) comprises GHSR or its salts, partial peptides of the GHSR or their salts, GHSR-containing cells or membrane fractions of the GHSR-containing cells, and the polypeptide of the present invention.

The screening kit according to the present invention comprises, for example, the following:

1. Reagent for screening

1) Buffers for assay and washing

Hanks' Balanced Salt Solution (manufactured by Gibco) supplemented with 0.05% of bovine serum albumin (manufactured by Sigma).

The buffers may be sterilized by filtration through a membrane filter with a 0.45 μm pore size and stored at 4°C, or may be prepared at use.

2) Standard GHSR preparation

GHSR-expressed CHO cells are subcultured at 5 x $10^5$ cells/well on a 12-well plate followed by culturing at 37°C under a 5% $CO_2$ and 95% air for 2 days.

3) Labeled ligand

Ligand labeled with [$^3$H], [$^{125}$I], [$^{14}$C], [$^{35}$S], etc.
The product dissolved in a suitable solvent or buffer is stored at 4°C or at -20°C, which will be diluted at use to 1 μM with a buffer for the assay.

4) Standard ligand solution

The polypeptide of the present invention is dissolved in PBS containing 0.1% of bovine serum albumin (manufactured by Sigma) to make the volume 1 mM and then stored at -20°C.

2. Method for assay

1) Cells are cultured in a 12-well tissue culture plate to express the GHSR. After washing the cells twice with 1 ml of the buffer for assay, 490 μl of the buffer for assay is added to each well.
2) After 5 μl of a test compound solution of $10^{-3}$ to $10^{-10}$ M is added, 5 μl of the labeled peptide of the present invention is added to the system followed by incubation at room temperature for an hour. To determine the amount of the non-specific binding, 5 μl of the $10^{-3}$ M polypeptide of the present invention is added to the system, instead of the test compound.
3) The reaction mixture is removed and the cells are washed three times with 1 ml each of the washing buffer. The labeled polypeptide of the present invention bound to the cells is dissolved in 0.2N NaOH-1% SDS and mixed with 4 ml of a liquid scintillator A (manufactured by Wako Pure Chemical).
4) Radioactivity is measured using a liquid scintillation counter (manufactured by Beckman) and percent of the maximum binding (PMB) is calculated in accordance with the following equation [1]:

Equation [1] $$PMB = [(B - NSB)/(B_0 - NSB)] \times 100$$

wherein:

PMB : percent of the maximum binding
B : value when a specimen is added
NSB : non-specific binding
$B_0$ : maximum binding

The compound or salts thereof obtainable by the screening methods or by the screening kits of the present invention is the compound that alters the binding property between the polypeptide of the present invention and GHSR (inhibits or promotes the binding), specifically, a compound or salts thereof having a GHSR-mediated cell-stimulating activity (so-called GHSR agonists) or a compound having no cell-stimulating activity (so-called GHSR antagonists). Examples of the compound include a peptide, a protein, a non-peptide compound, a synthetic compound, a fermentation product, etc. and the compound may be either novel or known compound.
To determine if the compound is the GHSR agonists or antagonists, the following method (i) or (ii) is available.

(i) The binding assay recited in the screening methods 1) through 3) *supra* is performed to obtain the compound that alters the binding property between the polypeptide of the present invention and GHSR (especially inhibits the binding) followed by assay for the compound to determine if the compound has the GHSR-mediated cell-stimulating activity described above. The compound having the cell-stimulating activity or its salts are GHSR agonists, whereas the compound having no such activity or its salts are GHSR antagonists.
(ii) (a) A test compound is brought into contact with GHSR-containing cells to assay the GHSR-mediated cell-stimulating activity. The compound having the cell-stimulating activity or its salts are GHSR agonists.
(b) The GHSR-mediated cell-stimulating activity is measured both when a compound (e.g., the polypeptide of the present invention, a GHSR agonist, etc.) that activates GHSR is brought into contact with a GHSR-containing cell and when the compound that activates GHSR and a test compound are brought into contact with the GHSR-containing cell and comparison is made on the cell-stimulating activity between the two cases. The compound capable of reducing the cell-stimulating activity by the GHSR-activating compound or its salts are GHSR antagonists.

The GHSR agonists exhibit activities similar to the physiological activities of the polypeptide of the present invention and are thus useful as safe and low toxic drugs, likewise the polypeptide of the present invention.
To the contrary, since the GHSR antagonists can suppress the physiological activities that the polypeptide of the present invention possesses, they are useful as safe and low toxic drugs for suppressing the receptor activity.
The polypeptide of the present invention participates in the GH secretion promoting action, etc. Thus, the GHSR agonists may be used as a therapeutic and prophylactic agent for diseases caused by deficiency of GH, e.g., pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, or the like. On the other hand, the GHSR antagonists may be used as a therapeutic and prophylactic agent for diseases caused by oversecretion of GH, e.g., acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, or the like.
The salts of the compound obtainable by using the screening methods or kits described above are, for example, pharmaceutically acceptable salts. Examples of the salts include salts with inorganic bases, salts with organic bases, salts with inorganic acids, salts with organic acids and salts with basic or acidic amino acids, and the like.
Preferred examples of the salts with inorganic bases include alkali metal salts such as sodium salts, potassium salts, etc.; alkaline earth metal salts such as calcium salts, magnesium salts, etc.; and aluminum salts, ammonium salts, and the like.
Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.
Preferred examples of the salts with inorganic acids include salts with hydrochloric acid, hydrobromic acid, sulfuric acid, phosphoric acid, etc.
Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.
Preferred examples of the salts with basic amino acids include salts with arginine, lysine, ornithine, etc. Preferred examples of the salts with acidic amino acids include salts with aspartic acid, glutamic acid, etc.
Where the compound or salts thereof obtainable by the screening methods or kits of the present invention are used as the drugs described above, pharmaceutical preparations may be prepared and administered as in the case of using the polypeptide of the present invention as a drug.

(4) Manufacture of antibodies to the polypeptide of the present invention or antisera

Antibodies (e.g., polyclonal antibodies, monoclonal antibodies) to the polypeptide of the present invention, or antisera can be manufactured by publicly known methods for manufacturing antibodies or antisera, using the polypeptide of the present invention as an antigen.
For example, polyclonal antibodies can be manufactured by the method described below.

[Preparation of polyclonal antibody]

A polyclonal antibody to the polypeptide of the present invention can be manufactured by publicly known methods or modifications thereof. The polyclonal antibody can be manufactured, for example, by producing the complex of an immunogen (a polypeptide antigen, etc.) and a carrier protein, immunizing a warm-blooded animal (e.g., mammals (e.g., rabbit, sheep, goat, rat, mouse, guinea pig, bovine, horse, swine), birds (e.g., chicken, dove, duck, goose, quail), etc.) as in the method for manufacturing a monoclonal antibody described below, collecting the product containing the antibody to the polypeptide of the present invention from the immunized animal and then separating and purifying the antibody.
In the complex of an immunogen and a carrier protein used to immunize a mammal, a type of the carrier protein and a mixing ratio of the carrier to the hapten (the polypeptide of the present invention) may be any type and in any ratio, as long as the antibody is efficiently produced to the hapten immunized by crosslinking to the carrier. For example, bovine serum albumin, bovine thyroglobulin, Keyhole limpet hemocyanin, etc. is coupled to the hapten in a carrier-to-hapten weight ratio of approximately 0.1 to 20, preferably about 1 to about 5.
A variety of condensation agents can be used for coupling of the carrier to the hapten. Glutaraldehyde, carbodiimide, maleimide activated ester, activated ester reagents containing thiol group or dithiopyridyl group and the like are used for the coupling.

The condensation product is administered to the warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to about 6 weeks and about 3 to about 10 times in total.
The polyclonal antibody can be collected from the blood, ascites, etc., preferably from the blood of the mammal immunized by the method described above.
The antibody titer to the polypeptide of the present invention in antisera can be assayed by the same procedure as that for determining the antibody titer in hybridoma culture supernatant described hereinafter. The separation and purification of the antibody can be made, following the method for the separation and purification of immunoglobulins performed as in the separation and purification of monoclonal antibodies described below.
A monoclonal antibody can also be manufactured according to the method described below.

[Preparation of monoclonal antibody]

(a) Preparation of monoclonal antibody-producing cells

The polypeptide of the present invention is administered to warm-blooded animals (for example, mammals (e.g., rabbit, sheep, goat, rat, mouse, guinea pig, bovine, horse, swine), birds (e.g., chicken, dove, duck, goose, quail) and the like) either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every 2 to 6 weeks and 2 to 10 times in total.
In preparing monoclonal antibody-producing cells, the aforesaid warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells to give monoclonal antibody-producing hybridomas. The antibody titer in antisera may be assayed, for example, by reacting the labeled polypeptide of the present invention, which will be described later, with the antiserum followed by assaying the activity of the labeling agent bound to the antibody. The fusion may be carried out, for example, by the known method by Koehler and Milstein [Nature, 256, 495, (1975)]. Examples of the fusion promoter used include polyethylene glycol (PEG), Sendai virus, etc., of which PEG is preferably employed.
Examples of the myeloma cells are NS-1, P3U1, SP2/0, AP-1, etc. In particular, P3U1 is preferably employed. A preferred ratio of the count of the antibody-producing cells (spleen cells) used to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, efficient cell fusion can be carried out.
Various methods can be used for the screening of a monoclonal antibody-producing hybridoma to the polypeptide of the present invention. Examples of such methods include a method which comprises adding the supernatant of a hybridoma to a solid phase (e.g., microplate) adsorbed with the polypeptide of the present invention as an antigen directly or together with a carrier, adding an anti-immunoglobulin antibody (where mouse cells are used for the cell fusion, an anti-mouse immunoglobulin antibody is used) labeled with a radioactive substance or an enzyme, or Protein A and detecting the monoclonal antibody to the polypeptide of the present invention bound to the solid phase; a method which comprises adding the supernatant of hybridoma to a solid phase adsorbed with an anti-immunoglobulin antibody or Protein A, adding the polypeptide of the present invention labeled with a radioactive substance or an enzyme and detecting the monoclonal antibody against the polypeptide of the present invention bound to the solid phase, and so on.
The monoclonal antibody to the polypeptide of the present invention can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like may be used for the selection and growth medium. The cultivation is carried out generally at 20 to 40°C, preferably at about 37°C, for about 5 days to about 3 weeks, preferably 1 to 2 weeks, normally in 5% $CO_2$. The antibody titer of the culture supernatant of the hybridoma can be determined as in the assay for the antibody titer to the polypeptide of the present invention in the antisera described above.

(b) Purification of monoclonal antibody

The monoclonal antibody to the polypeptide of the present invention can be separated and purified, as applied to conventional separation and purification of polyclonal antibodies, according to the methods for separation and purification of immunogloblins [e.g., salting-out, alcohol precipitation, isoelectric point precipitation, electrophoresis, adsorption/desorption with ion exchangers (e.g., DEAE), ultracentrifugation, gel filtration, or a specific purification method which comprises collecting only an antibody by means of an antigen-binding solid phase or, an activated adsorbent such as Protein A or Protein G and dissociating the binding to obtain the antibody].
The antibodies to the polypeptide of the present invention manufactured by the methods (a) and (b) described above are capable of specifically recognizing the polypeptide of the present invention, respectively. Thus, these antibodies can be used for quantification of the polypeptide of the present invention in a test sample fluid, in particular, for quantification by the sandwich immunoassay.
That is, the present invention provides:

(i) a method for quantification of the polypeptide of the present invention in a test sample fluid, which comprises competitively reacting antibodies that are reactive with the polypeptide of the present invention, a test sample fluid and the labeled polypeptide of the present invention, and measuring the ratio of the labeled polypeptide of the present invention bound to the antibodies; and,
(ii) a method for quantification of the polypeptide of the present invention in a test sample fluid, which comprises reacting the test sample fluid simultaneously or continuously with an antibody immobilized on a carrier and a labeled antibody, and then measuring the activity of the labeling agent on the immobilized carrier, wherein one antibody is capable of recognizing the N-terminal region of the polypeptide of the present invention and another antibody is capable of recognizing the C-terminal region of the polypeptide of the present invention.

The polypeptide of the present invention may be assayed using the monoclonal antibody capable of recognizing the polypeptide of the present invention. In addition, the monoclonal antibody may also be used for detection by means of tissue staining, etc. For these purposes, the antibody molecule per se may be used or $F(ab')_2$, Fab' or Fab fractions of the antibody molecule may be used as well. There is no particular limitation to the assay method using the antibody of the present invention. Any method may be used as far as it relates to a method in which the amount of an antibody, an antigen or an antibody-antigen complex can be detected by a chemical or physical means, depending on or corresponding to the amount of antigen (e.g., the amount of the polypeptide) in a test sample fluid to be assayed, and then calculated using a standard curve prepared by a standard solution containing a known amount of the antigen. Advantageously used are, for example, nephrometry, competitive assay, immunometric assay and sandwich assay. In terms of sensitivity and specificity, the sandwich assay method, which will be described later, is particularly preferred for use.
Examples of the labeling agent used in the assay method using a labeling substance are radioisotopes, enzymes, fluorescent substances, luminescent substances, etc. Examples of the radioisotope are [$^{125}I$], [$^{131}I$], [$^{3}H$], [$^{14}C$], etc. Preferred examples of the enzyme are those that are stable and have a high specific activity, including β-galactosidase, β-glucosidase, an alkaline phosphatase, a peroxidase, malate dehydrogenase, etc. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may be used as well for binding between an antibody or antigen and a labeling agent.
Upon immobilization of antigens or antibodies, physical adsorption may be used. Alternatively, chemical binding that is conventionally used to immobilize proteins or enzymes may be used as well. Examples of the carrier include insoluble polysaccharides such as agarose, dextran, cellulose, etc.; synthetic resins such as polystyrene, polyacrylamide, silicone, etc.; glass; and the like.
In the sandwich assay method, a test sample fluid is reacted with an immobilized anti-polypeptide antibody (first reaction), then reacted with another labeled anti-polypeptide antibody (second reaction) and the activity of the labeling agent on the immobilized carrier is measured, whereby the amount of the polypeptide in the test sample fluid can be quantified. The first and second reactions may be carried out in a reversed order or simultaneously or they may be conducted with an interval.
The type of the labeling agent and the method for immobilization may be similar to those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.
In the method for assaying the polypeptide of the present invention by the sandwich method according to the present invention, antibodies wherein the binding sites to the polypeptide of the present invention are different from one another are preferably used as the anti-polypeptide antibody used for the first and the second reactions. Thus, the antibodies used in the first and the second reactions are those wherein, when the antibody used in the second reaction recognizes the C-terminal region of the polypeptide of the present invention, the antibody recognizing the site other than the C-terminal regions, e.g., recognizing the N-terminal region, is preferably used in the first reaction.

The antibody to the polypeptide of the present invention may be used in an assay system other than the sandwich method, such as a competitive method, an immunometric method, a nephrometry, etc. In a competitive method, an antigen in the test sample fluid and a labeled antigen are made to react with an antibody in a competitive manner, then an unreacted labeled antigen (F) and a labeled antigen binding with an antibody (B) are separated (B/F separation) and the labeled amount of any of B and F is measured, whereby the amount of the antigen in the test sample fluid is quantified. In the methods for such reactions, there are a liquid phase method, in which a soluble antibody is used as the antibody and the B/F separation is conducted by polyethylene glycol, a second antibody to the said antibody, etc. is used; and a solid phase method, in which an immobilized antibody is used as the first antibody or, a soluble antibody is used as the first antibody while an immobilized antibody is used as the second antibody.

In the immunometric method, an antigen in the test sample fluid and an immobilized antigen are subjected to a competitive reaction with a given amount of a labeled antibody followed by separating into solid and liquid phases; or the antigen in the test sample fluid is reacted with an excess amount of labeled antibody, then an immobilized antigen is added to bind an unreacted labeled antibody to the solid phase and the solid phase is separated from the liquid phase. After that, the labeled amount of any of the phases is measured to determine the amount of the antigen in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, a laser nephrometry utilizing laser scattering can be suitably used.

In applying each of those immunoassays to the assay method for the present invention, it is not necessary to set up any special condition, operation, etc. The assay system for the polypeptide of the present invention may be constructed in addition to conditions or operations conventionally used for each of the methods, taking into account the technical consideration of one skilled in the art. For the details of such conventional technical means, a variety of reviews, reference books, etc. may be referred to [for example, Hiroshi Irie (ed.): "Radioimmunoassay" (published by Kodansha, 1974)]; Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.].

As described above, the polypeptide of the present invention can be quantified with high sensitivity, using the antibody to the polypeptide of the present invention.

By quantifying the polypeptide of the present invention in a test sample fluid, diagnosis of diseases in which the polypeptide of the present invention participates can be conducted. Examples of such diseases, with which the polypeptide of the present invention is associated, are pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, etc. A test sample fluid can be prepared from mammals to be tested (e.g., human, rabbit, sheep, goat, rat, mouse, guinea pig, bovine, horse, swine) by publicly known methods. Examples of the test sample fluid include blood, lymph, urine, etc.

The antisense DNA having a complementary or substantially complementary base sequence to the DNA encoding the polypeptide of the present invention can be any antisense DNA, as long as it possesses a complementary or substantially complementary base sequence to the DNA of the present invention and capable of suppressing expression of the DNA.

The base sequence substantially complementary to the DNA encoding the polypeptide of the present invention may, for example, be a base sequence having at least about 70% homology, preferably at least about 80% homology, more preferably at least about 90% homology and most preferably at least about 95% homology, to the full-length base sequence or partial base sequence of the complementary base sequence to the DNA encoding the polypeptide of the present invention. Particularly in the entire base sequence of the complementary strand to the DNA encoding the polypeptide of the present invention, preferred are antisense DNAs having at least about 70% homology, preferably at least about 80% homology, more preferably at least about 90% homology and most preferably at least about 95% homology, to the complementary strand of the base sequence part encoding the N-terminal region of the polypeptide of the present invention (e.g., the base sequence around the initiation codon, etc.). These antisense DNAs can be synthesized using a publicly known DNA synthesizer, etc.

These antisense DNAs have the activity capable of suppressing the expression of the DNA encoding the polypeptide of the present invention, and can thus be used as therapeutic/prophylactic agents for diseases associated with excessive secretion of GH, e.g., acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, etc.

(5) Gene diagnostic agent

By using the DNA encoding the polypeptide of the present invention, e.g., as a probe, an abnormality (gene abnormality) of the DNA or mRNA encoding the polypeptide of the present invention in human or other warm-blooded animal (e.g., rat, mouse, guinea pig, rabbit, chicken, sheep, swine, bovine, horse, cat, dog, monkey, etc.) can be detected, and is thus useful as a gene diagnostic agent for the damage to the DNA or mRNA, its mutation, or its decreased expression, or promoted expression or overexpression of the DNA or mRNA.

The gene diagnosis described above using the DNA encoding the polypeptide of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), or the like.

In case that decreased expression is detected by, e.g., the Northern hybridization, it is highly likely to suffer from diseases associated with deficiency of GH, e.g., pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, etc. On the other hand, where an promoted expression is detected by the Northern hybridization, it is highly likely to suffer from diseases caused by overexpression of GH, e.g., acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent and insulin-independent diabetes mellitus, etc.

(6) Preparation of non-human animal bearing the DNA encoding the polypeptide of the present invention

Transgenic non-human mammals capable of expressing the polypeptide of the present invention can be prepared using the DNA encoding the polypeptide of the present invention. Examples of non-human mammals include mammals (e.g., rats, mice, rabbits, sheep, swine, bovine, cats, dogs, monkeys, and the like) (hereinafter merely referred to as animal).

To introduce the DNA encoding the polypeptide of the present invention into a target animal, it is generally advantageous to employ the DNA as a gene construct ligated downstream a promoter capable of expressing the DNA in an animal cell. For example, when the mouse-derived DNA of the present invention is introduced, for example, the gene construct, in which the DNA is ligated downstream various promoters capable of expressing the DNA of the present invention derived from an animal that is highly homologous to the DNA of the present invention, is microinjected to mouse fertilized ova. Thus, the DNA-introduced animal capable of producing a high level of the protein, etc. of the present invention can be produced. As the promoters, there may be used, e.g., a virus-derived promoter and a ubiquitous expression promoter such as metallothionein, etc.

The introduction of the DNA encoding the polypeptide of the present invention into the fertilized egg cell stage secures the presence of the DNA in all germ and somatic cells in the target animal. The presence of the polypeptide, etc. of the present invention in the germ cells in the DNA-introduced animal means that all germ and somatic cells contain the polypeptide, etc. of the present invention in all progenies of the animal. The progenies of the animal that took over the gene contain the polypeptide, etc. of the present invention in all germ and somatic cells.

The transgenic animal to which the DNA encoding the polypeptide of the present invention has been introduced can be subjected to mating and breeding for generations under common breeding circumstance, as the DNA-carrying animal, after confirming that the gene can be stably retained. Moreover, male and female animals having the desired DNA are mated to give a homozygote having the transduced gene in both homologous chromosomes and then the male and female animals are mated so that such breeding for generations that progenies contain the DNA can be performed.

The transgenic animal to which the DNA encoding the polypeptide of the present invention has been introduced is useful as the animal for screening of the agonists or antagonists to the polypeptide, etc. of the present invention, since the polypeptide, etc. of the present invention is abundantly expressed.

The transgenic animal to which the DNA encoding the polypeptide of the present invention has been introduced can also be used as the cell sources for tissue culture. The polypeptide, etc. of the present invention can be analyzed by, for example, direct analysis of DNA or RNA in the tissues in mice, into which the DNA encoding the polypeptide of the present invention has been introduced, or by analysis of the tissues containing the polypeptide of the present invention expressed from the gene. Cells from tissues containing the polypeptide, etc. of the present invention are cultured by the standard tissue culture technique. Using these cells, the function of the cells from the tissues that are generally difficult to culture, for example, cells derived from the brain and peripheral tissues can be studied. Using these cells it is also possible to screen pharmaceuticals, for example, that enhance the function of various tissues. Where a highly expressing cell line is available, the polypeptide, etc. of the present invention can be isolated and purified from the cell line.

In the specification and drawings, when bases, amino acids, etc. are shown by abbreviations, the codes of bases and amino acids are denoted in accordance with the IUPAC-IUB Commission on Biochemical Nomenclature or by the codes conventionally used in the art, examples of which are shown below. With respect to amino acids that may have their optical isomers, L form is given, unless otherwise indicated.

| | |
|---|---|
| DNA : | deoxyribonucleic acid |
| cDNA : | complementary deoxyribonucleic acid |
| A: | adenine |
| T : | thymine |
| G : | guanine |
| C : | cytosine |
| Y : | thymine or cytosine |
| N : | thymine, cytosine, adenine or guanine |
| R : | adenine or guanine |
| M: | cytosine or adenine |
| W : | thymine or adenine |
| S : | cytosine or guanine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| TFA : | trifluoroacetic acid |
| EIA : | enzyme immunoassay |
| Gly or G: | glycine |
| Ala or A: | alanine |
| Val or V: | valine |
| Leu or L: | leucine |
| Ile or I: | isoleucine |
| Ser or S: | serine |
| Thr or T: | threonine |
| Cys or C: | cysteine |
| Met or M: | methionine |
| Glu or E: | glutamic acid |
| Asp or D: | aspartic acid |
| Lys or K: | lysine |
| Arg or R: | arginine |
| His or H: | histidine |
| Phe or F: | phenylalanine |
| Tyr or Y: | tyrosine |
| Trp or W: | tryptophan |
| Pro or P: | proline |
| Asn or N: | asparagine |
| Gln of Q : | glutamine |
| pGlu : | pyroglutamic acid |
| Me: | methyl group |
| Et : | ethyl group |
| Bu : | butyl group |

| | |
|---|---|
| Ph : | phenyl group |
| Tc : | thiazoline-4(R)-carboxamide group |
| Bom : | benzyloxymethyl |
| NMP : | N-methylpyrrolidone |
| PAM : | phenylacetamidomethyl |

The substituents, protecting groups and reagents which are frequently used in the present specification are denoted by the following symbols.

| | |
|---|---|
| Tos: | p-toluenesulfonyl |
| HONB : | N-hydroxy-5-norbornene-2,3-dicarboximide |
| Bzl : | benzyl |
| Z: | benzyloxycarbonyl |
| Br-Z : | 2-bromobenzyloxycarbonyl |
| Cl-Z : | 2-chlorobenzyloxycarbonyl |
| Boc : | t-butoxycarbonyl |
| HOBt : | 1-hydroxybenztriazole |
| DCC : | N,N'-dichlorohexylcarbodiimide |
| TFA : | trifluoroacetic acid |
| Fmoc : | N-9-fluorenylmethoxycarbonyl |
| DNP : | dinitrophenol |
| Bum : | t-butoxymethyl |
| Trt : | trityl |
| MeBzl : | 4-methylbenzyl |
| CHO : | formyl |
| NMP : | N-methylpyrrolidone |
| OcHex : | cyclohexyl ester |

The sequence identification numbers in the sequence listing of the specification indicate the following sequences.

[SEQ ID NO: 1]

This shows the amino acid sequence encoding the ligand polypeptide of the present invention obtained in EXAMPLE 3 later described.

[SEQ ID NO: 2]

This shows the base sequence of the DNA encoding the amino acid sequence represented by SEQ ID NO: 1 (bovine type).

[SEQ ID NO: 3]

This shows the amino acid sequence encoding bovine type polypeptide obtained in EXAMPLE 5 later described.

[SEQ ID NO: 4]

This shows the base sequence of the DNA encoding the amino acid sequence represented by SEQ ID NO: 3 (bovine type).

[SEQ ID NO: 5]

This shows the amino acid sequence encoding human type polypeptide obtained in EXAMPLE 6 later described.

[SEQ ID NO: 6]

This shows the base sequence of the DNA encoding the amino acid sequence represented by SEQ ID NO: 5 (human type).

[SEQ ID NO: 7]

This shows the amino acid sequence encoding rat type polypeptide obtained in EXAMPLE 7 later described.

[SEQ ID NO: 8]

This shows the base sequence of the DNA encoding the amino acid sequence represented by SEQ ID NO: 7 (rat type).

[SEQ ID NO: 9]

This shows the base sequence of the DNA encoding the amino acid sequence represented by SEQ ID NO: 1 (human type).

[SEQ ID NO: 10]

This shows the sequence of 2-18 amino acids from the N-terminus of the amino acid sequence represented by SEQ ID NO: 7.

[SEQ ID NO: 11]

This shows the base sequence of the DNA encoding the amino acid sequence represented by SEQ ID NO: 10.

[SEQ ID NO: 12]

This shows the base sequence of primer bF used in EXAMPLE 5 later described.

[SEQ ID NO: 13]

This shows the base sequence of primer bR used in EXAMPLE 5 later described.

[SEQ ID NO: 14]

This shows the base sequence of primer hF used in EXAMPLE 6 later described.

[SEQ ID NO: 15]

This shows the base sequence of primer hR used in EXAMPLE 6 later described.

[SEQ ID NO: 16]

This shows the base sequence of primer rF used in EXAMPLE 7 later described.

[SEQ ID NO: 17]

This shows the base sequence of primer rR used in EXAMPLE 7 later described.

[SEQ ID NO: 18]

This shows the amino acid sequence encoding GHSR (Hum Ia) described in Howard et al., Science, vol. 273, p974-977 (1996).

[SEQ ID NO: 19]

This shows the amino acid sequence encoding GHSR (Pig Ia) described in Howard et al., Science, vol. 273, p974-977 (1996).

[SEQ ID NO: 20]

This shows the amino acid sequence encoding GHSR (Hum Ib) described in Howard et al., Science, vol. 273, p974-977 (1996).

[SEQ ID NO: 21]

This shows the amino acid sequence encoding GHSR (Pig Ib) described in Howard et al., Science, vol. 273, p974-977 (1996).

[SEQ ID NO: 22]

This shows the base sequence of primer GHCF used in EXAMPLE 1 later described.

[SEQ ID NO: 23]

This shows the base sequence of primer GHR used in EXAMPLE 1 later described.

[SEQ ID NO: 24]

This shows the amino acid sequence encoding rat type GHSR.

[SEQ ID NO: 25]

This shows DNA encoding the amino acid sequence shown by SEQ ID NO: 24.

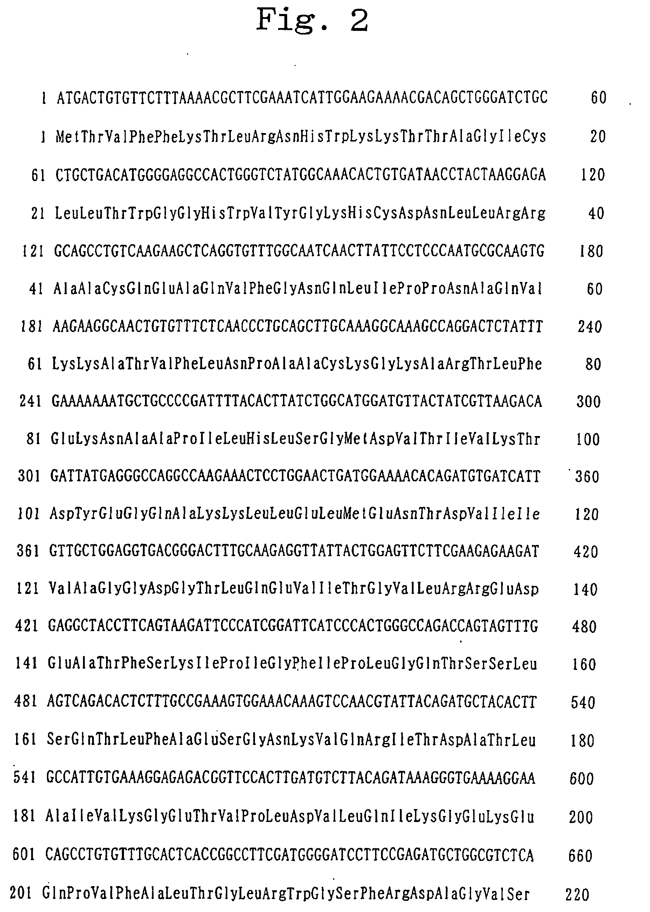

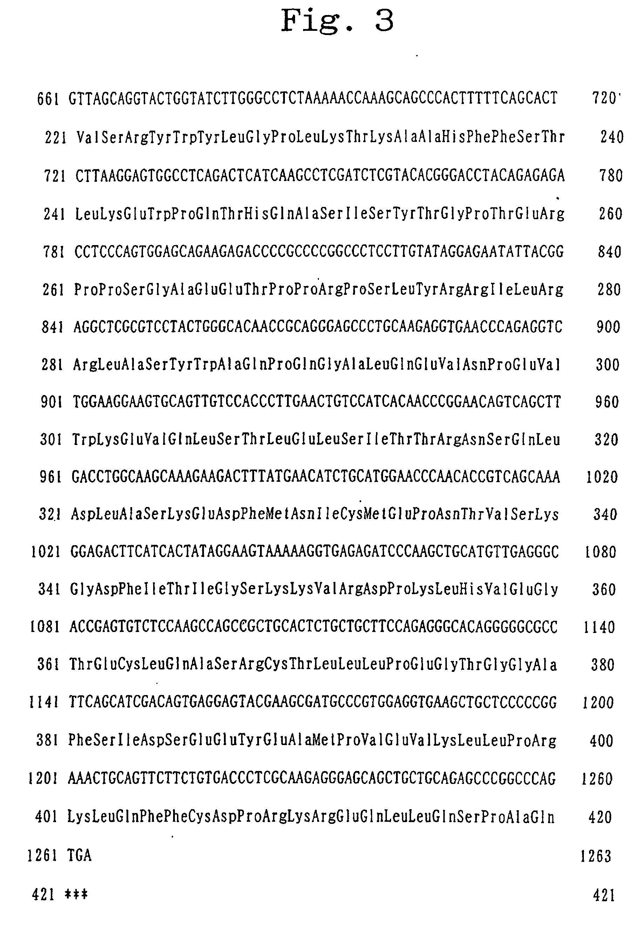

Transformant *Escherichia coli* JM109/pbKW1 bearing bovine type GHSR endogenous ligand gene (SEQ ID NO: 4, FIGS. 2 and 3), which was obtained in EXAMPLE 5 later described, was on deposit with the Ministry of International Trade and Industry, Agency of Industrial Science and Technology, National Institute of Bioscience and Human Technology (NIBH) as the Accession Number FERM BP-6961 on December 6, 1999 and with Institute for Fermentation (IFO) as the Accession Number IFO 16326 on October 27, 1999.

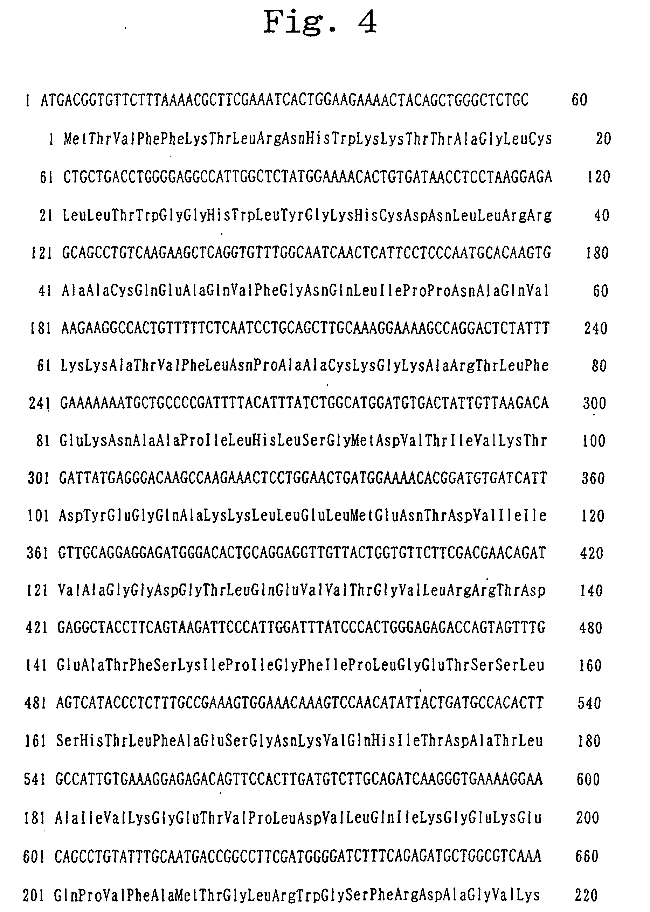

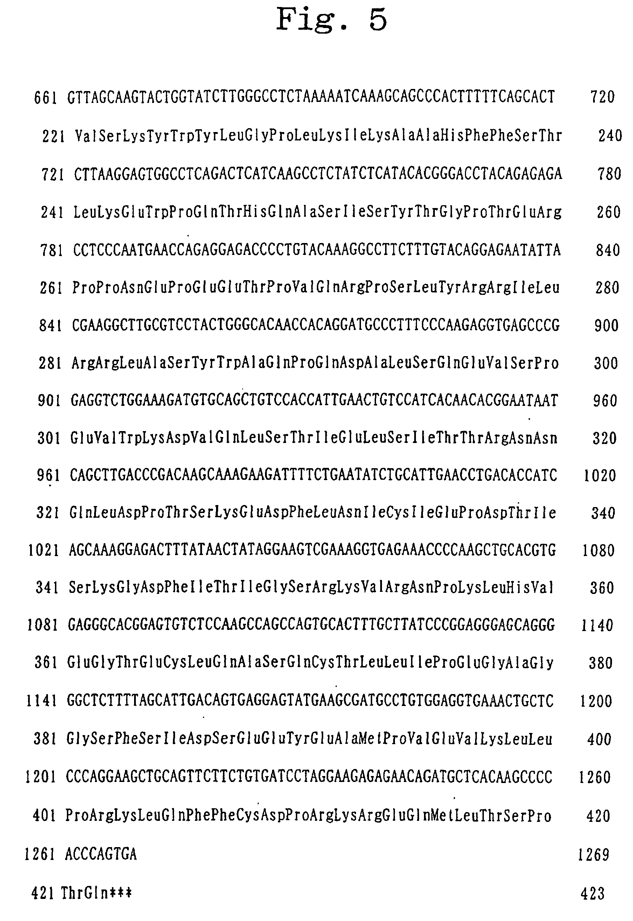

Transformant *Escherichia coli* JM109/phKW9 bearing human type GHSR endogenous ligand gene (SEQ ID NO: 6, FIGS. 4 and 5), which was obtained in EXAMPLE 6 later described, was on deposit with the Ministry of International Trade and Industry, Agency of Industrial Science and Technology, National Institute of Bioscience and Human Technology (NIBH) as the Accession Number FERM BP-6963 on December 6, 1999 and with Institute for Fermentation (IFO) as the Accession Number IFO 16327 on October 27, 1999.

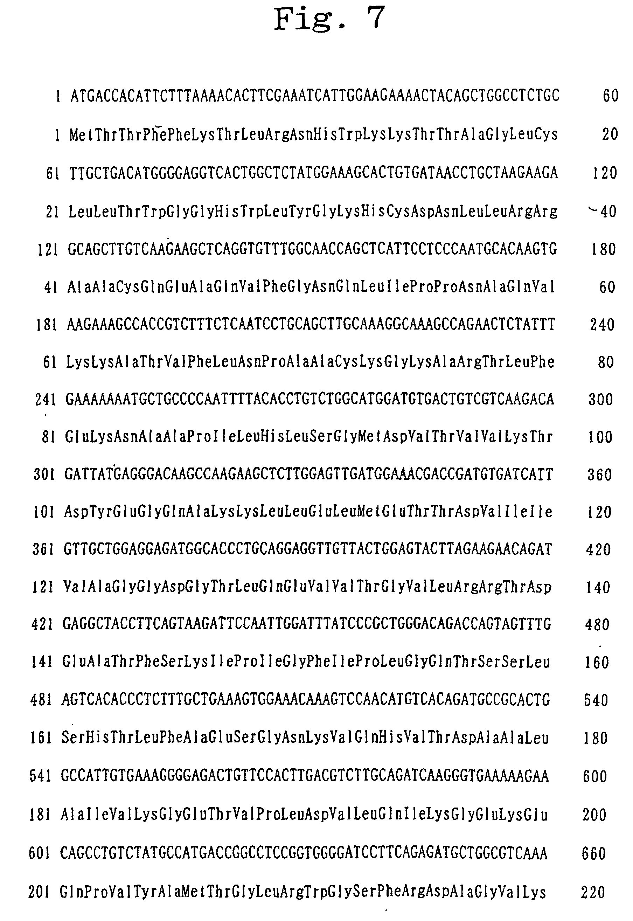

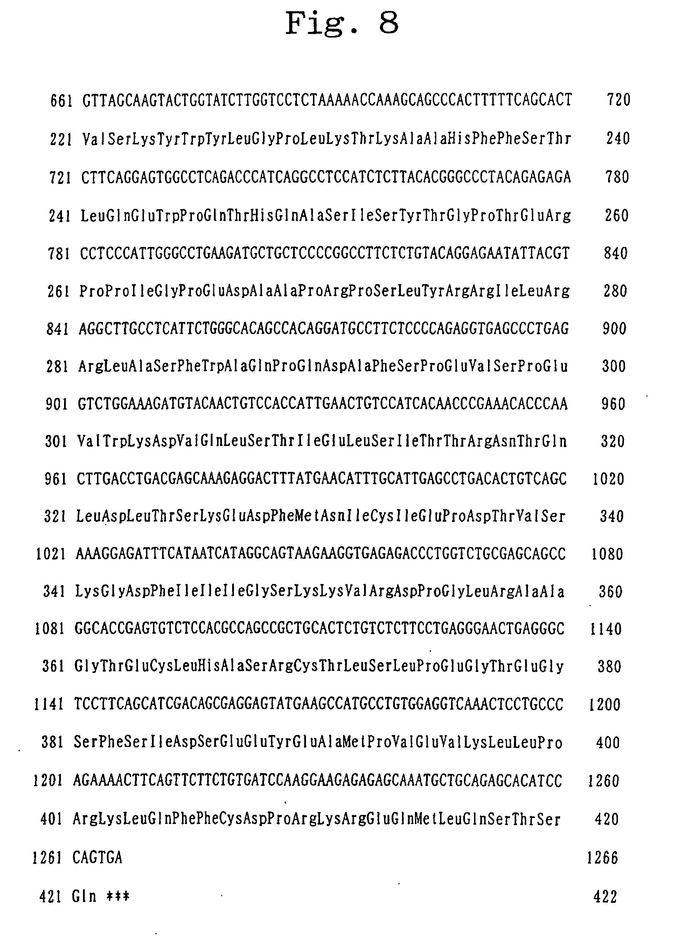

Transformant *Escherichia coli* JM109/prKW4 bearing rat type GHSR endogenous ligand gene (SEQ ID NO: 8, FIGS. 7 and 8), which was obtained in EXAMPLE 7 later described, was on deposit with the Ministry of International Trade and Industry, Agency of Industrial Science and Technology, National Institute of Bioscience and Human Technology (NIBH) as the Accession Number FERM BP-6962 on December 6, 1999 and with Institute for Fermentation (IFO) as the Accession Number IFO 16328 on October 27, 1999.

EXAMPLES

The present invention is described below in more detail with reference to EXAMPLES, but not intended to limit the scope of the present invention thereto.

EXAMPLE 1 Establishment of GHSR-expressing CHO cells

Rat type GHSR (growth hormone secretagogue receptor) gene was acquired as follows. Based on the sequence of rat GHSR type 1A reported in Molecular Endocrinology, 11 (4), 415-423 (1997), the following two synthetic DNAs were synthesized.

GHCF: 5'-GTCGACCATGTGGAACGCGACCCCAGCGAGGAG-3'

GHR: 5'-GCTAGCGGAGAGATGGGATGTGCTGTCATGT-3'

Using these synthetic DNAs, PCR (polymerase chain reaction) was carried out to acquire the GHSR gene from rat hypothalamus-derived cDNA. The reaction solution for PCR containing 2 µl (0.2 ng poly(A)$^+$ RNA-derived) rat hypothalamus-derived cDNA solution, 1 µl (10 µM) of GHCF, 1 µl (10 µM) of GHR, 5 µl of 10 x buffer solution attached, dNTPs (1 mM), 1 µl of Klen Taq (Clontech Inc.) was made 50 µl in total by adding 35 µl of distilled water. Using Thermal Cycler 9600 (Perkin-Elmer Co.), PCR was carried out in the reaction solution. The conditions for PCR were as follows: the reaction mixture was heated at 95°C for 2 minutes for denaturation and then subjected to 35 cycles of 98°C for 10 seconds and 68°C for 90 seconds. Using an aliquot of the amplification product, it was confirmed by electrophoresis that the PCR product of about 1.1 kb was amplified. Then, the PCR product was subcloned using a TA Cloning Kit (Invitrogen, Inc.). After plasmid was extracted from E. coli acquired through subcloning using an automatic plasmid extracting machine (Kurabo Co., Ltd.), the base sequence of the inserted fragment was determined and identified to be the same GHSR cDNA as reported in the journal supra. Next, the plasmid was digested with restriction enzymes SalI and NheI to give GHSR cDNA fragment of about 1.1 kb. Expression vector pAKKO-111H for an animal cell was further digested with restriction enzymes SalI and NheI, which recognition sites present in the multicloning site, and the digestion product was subjected to electrophoresis to recover the vector part. The fragment of rat GHS receptor cDNA and the expression vector, which were prepared by the foregoing procedures, were ligated through ligation, and E. coli JM109 was transformed to give E. coli JM109/pAKKOGHSR. The transformant E. coli JM109/pAKKOGHSR was further cultured and the plasmid pAKKOGHSR DNAs were mass-produced.

After a 20 µg aliquot of the plasmid DNAs was dissolved in 1 ml of physiological buffered saline (PBS), the solution was charged in a gene transfer vial (Wako Pure Chemical Ind.) and vortexed well with a vortex mixer to form liposomes containing the DNA. CHO dhfr$^-$ cells were seeded in a Petri's dish of 35 mm diameter for cell culture in 1 to 2 x $10^6$ followed by incubation for 20 hours. After a fresh medium was exchanged for the culture medium, the liposome liquid was dropped into each Petri's dish in an amount (25 µl) corresponding to 0.5 µg of the DNA, followed by incubation for 16 hours to introduce the plasmid DNA. After the medium was replaced with a fresh medium and further incubation was performed for one day, a selection medium was replaced for the medium followed by incubation for 3 days. Finally, the cells were digested with trypsin and the dispersed cells were seeded in a screening medium (minimum essential medium free of deoxyribonucleosides and ribonuclreosides, alpha medium supplemented with 10% dialyzed bovine serum) in a low cell density for transformant selection. Since it was possible to grow only the transformants in the screening medium, the transformants were subcloned repeatedly for screening to establish CHO-GHSR cells.

It was likely that some of the CHO-GHSR cells thus acquired would be different in the number of the GHSR receptor expressed on the cells. When the cell capable of highly producing the receptor is cloned from such a cell population, an assay can be performed stably in a high sensitivity. For this reason, GHRP-6 that is an artificial peptide capable of acting on GHSR was used as a standard specimen and a highly reactive clone was selected in terms of arachidonic acid metabolite releasing activity as an index. As the highly reactive clone thus acquired, the CHO-GHSR-23 cell was established and the established cell was used for survey of its endogenous ligand.

EXAMPLE 2 Detection of the activity to specifically stimulate CHO-GHSR-23 cells in a tissue extract

From bovine hypothalamus, the extract was prepared basically by the procedures described below and stored in a refrigerator, which was used as a specimen for screening the cell stimulating activity.

The bovine hypothalamus was ground, boiled and extracted with 1M acetic acid. After 0.05% TFA (trifluoroacetic acid) was added to the supernatant separated after centrifugation followed by adsorption onto C18 column, elution was stepwise carried out with acetonitrile ($CH_3CN$) of 10%, 30% and 50% concentrations. After adjusting to 20 mM ammonium acetate ($CH_3COONH_4$)-10% acetonitrile (pH 4.7), the individual eluates were adsorbed onto CM Sepharose cation exchange column (HiPrep CM Sepharose FF, Pharmacia) followed by elution with sodium chloride (NaCl) of 100 mM, 200 mM, 500 mM and 1000 mM. Each fraction was diluted with 3 volumes of acetone, centrifuged for deproteination, and applied to 3 ml of the reverse phase column RESOURCE RFC (Pharmacia) for adsorption. After fractionation on a concentration gradient of 10 to 30% acetonitrile, each fraction was lyophilized.

The cell stimulating activity was determined in terms of the activity of releasing arachidonic acid metabolites from the cells. A 0.5 ml aliquot of the dilution obtained by dispersing CHL-GHSR-23 cells or control cells through trypsin digestion and diluting in a medium to 1.0 x $10^5$ cells/ml was dispensed in a 24-weel plate, and cultured overnight. A medium containing 1/200 [$^3$H]-arachidonic acid (NEN) was replaced for the medium followed by further incubation overnight. The culture thus obtained was used for assaying the activity. The cells were washed 3 times with an assay buffer (minimum essential medium free of deoxyribonucleosides and ribonuclreosides, alpha medium supplemented with 0.1% bovine serum albumin, 15 mM HEPES, pH 7.3) and then preincubated at 37°C for 30 minutes. After further washing twice, 0.5 ml of a sample was added to each well followed by incubation at 37°C for 30 minutes. The radioactivity of [$^3$H]-arachidonic acid metabolites released into the supernatant was measured with a scintillation counter.

A sample prepared from bovine hypothalamus by the procedures above was dissolved in a small quantity of DMSO (dimethylsulfoxide) and the solution was diluted with an assay buffer. The dilution was used as a specimen for determining the activity for releasing arachidonic acid metabolites from the CHO-GHSR-23 cells. As a result, the activity to specifically stimulate the CHO-GHSR-23 cells was detected in Fraction 8, as compared to the control cells.

EXAMPLE 3 Purification of the activity to specifically promote the release of arachidonic acid metabolites from the CHO-GHSR-23 cells using bovine hypothalamus

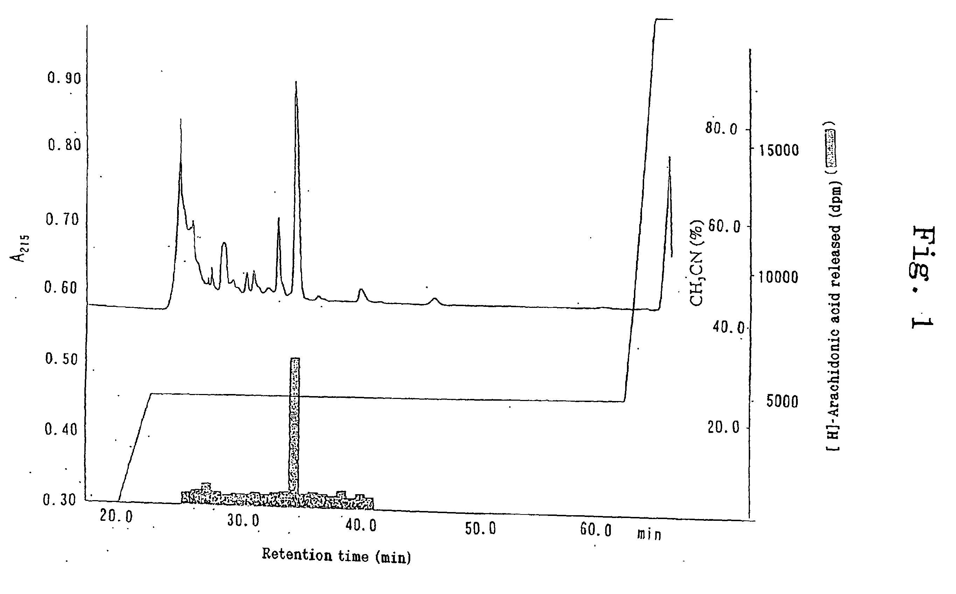

A representative example of purifying from bovine hypothalamus the active substances showing the arachidonic acid metabolite release activity specific to the CHO-GHSR-23 cells is described below. Bovine hypothalamus, 2 kg, was finely divided and boiled in 4.0 L of distilled water for 15 minutes. After quenching, 280 ml of acetic acid was added at a final concentration of 1.0 M and the mixture was homogenized using a Polytron (12 krpm, 6 mins.). The homogenate was stirred overnight and then centrifuged to separate the supernatant. The sediment was suspended in 2.0 L of 1.0M acetic acid, homogenized using a Polytron and centrifuged again to recover the supernatant. The supernatants were pooled, and TFA was added thereto at a final concentration of 0.05%. The mixture was applied to a reverse phase C18 column (Preparative C18 125Å 100 g; Waters), which had been equilibrated. After application of the supernatant, the column was washed with 600 ml of 0.05% TFA/d$H_2O$ (hereinafter d$H_2O$ refers to distilled water), and 3-gradient elution was carried out with 10%, 30% and 50% $CH_3CN$/0.1% TFA/d$H_2O$. To the fractions eluted with 30% $CH_3CN$/0.1% TFA/d$H_2O$, 2 volumes of 20 mM $CH_3COONH_4$/d$H_2O$, pH 4.7, were added, and the mixture was applied onto cation exchange column HiPrep CM Sepharose FF (20 ml) (Pharmacia). After washing the column with 20 mM $CH_3COONH_4$/10% $CH_3CN$/d$H_2O$, pH 4.7, 4-gradient elution was carried out with 100 mM, 200 mM, 500 mM and 1000 mM NaCl/20 mM $CH_3COONH_4$/10% $CH_3CN$/d$H_2O$, pH 4.7. The fraction eluted with 500 mM NaCl/20 mM $CH_3COONH_4$/10% $CH_3CN$/d$H_2O$, pH 4.7 was diluted with 500 mM NaCl/20 mM $CH_3COONH_4$/10% $CH_3CN$/d$H_2O$, pH 4.7 to 5-fold. The dilution was further diluted with 3 volumes of acetone, centrifuged for deproteination, and concentrated by evaporation. To the concentrated fraction was added TFA (final concentration of 0.05%). The mixture was applied to 3 ml of reverse phase column RESOURCE RPC (Pharmacia). Elution was carried out on a concentration gradient of 12.0 to 19.0% $CH_3CN$. As a result, the activity to specifically promote the release of arachidonic acid metabolites from the CHO-GHSR-23 cells was detected in the 16.5 to 17.0% $CH_3CN$ fraction. The active fraction eluted was again added to 3 ml of RESOURCE RPC, and elution was carried out on a concentration gradient of 14.0 to 18.0% $CH_3CN$. As a result, the arachidonic acid metabolite releasing activity from the CHO-GHSR-23 cells was detected in the 17.5% $CH_3CN$ fraction. This active fraction was diluted with 20 mM $CH_3COONH_4$/10% $CH_3CN$/d$H_2O$, pH 4.7, and the dilution was applied to 1 ml of cation exchange column RESOURCE S (Pharmacia). A concentration gradient fractionation of 300-800 mM NaCl/20 mM $CH_3COONH_4$/10% $CH_3CN$/d$H_2O$, pH 4.7 was carried out, and the fraction obtained was desalted to determine the sample activity using SepPak C18 column (Waters). As a result, the arachidonic acid metabolite releasing activity from the CHO-GHSR-23 cells was detected in the 600-640 mM NaCl fraction. These active fractions were added to reverse phase column Sephasil C8, 5 µm SC 2.1/10, and elution was carried out on a concentration gradient of 21.4-22.4% $CH_3CN$. As a result, the activity specific to release of arachidonic acid metabolites from the CHO-GHSR-23 cells was detected in the peak eluted with 21.7% $CH_3CN$ (FIG. 1).

EXAMPLE 4 Determination of amino acid sequences for the active substances to promote the arachidonic acid metabolite release activity specifically to the CHO-GHSR-23 cells purified from bovine hypothalamus

The amino acid sequence of the active substance having the activity to specifically promote the release of arachidonic acid metabolites from the CHO-GHSR-23 cells as purified in EXAMPLE 3 was determined. The fraction of peak activity identified to be identical by reverse phase column Sephasil C8, 5 µm SC 2.1/10 was lyophilized and analyzed for amino acid sequence with a peptide sequencer (PE Applied Biosystems Procise 491cLC). As a result, the sequence defined by SEQ ID NO: 1 was obtained.

EXAMPLE 5 Acquisition of endogenous ligand gene to bovine GHSR

To acquire a fragment encoding the full-length coding region of bovine GHSR endogenous ligand gene, the following 2 DNAs were synthesized.

bF : 5'-CAGCCGTGCAAATCCCTAAGAGAAGATGAC-3' (SEQ ID NO: 12)

bR : 5'-GCGGGGGACAGAGGAGGCCTTGGTTC-3' (SEQ ID NO: 13)

Using Marathon cDNA Amplification Kit (Clontech, Inc.) according to the manual attached, PCR was carried out using as a template cDNA synthesized from 1 µg of bovine hypothalamus poly(A)$^+$ RNA. The reaction solution for PCR contained 4 µl of cDNA solution (4 ng poly(A)$^+$ RNA-derived), 1 µl of dNTPs (10 mM), 0.5 µl of Klen Taq DNA polymerase (Clontech, Inc.), 10 x buffer attached to the 2.5 µl Klen Taq DNA polymerase and 16 µl of distilled water, to which 0.5 µl each of synthetic DNA bF and bR (10 µM, respectively) were further added to make the volume 25 µl in total. PCR was proceeded with the reaction solution using Thermal Cycler 9600. The reaction conditions were as follows: the reaction mixture was heated at 94°C for 2 minutes for denaturation and then subjected to 38 cycles of 98°C for 10 seconds and 68°C for 60 seconds. An aliquot of the PCR product was subjected to electrophoresis to confirm amplification of the PCR product of about 1.4 kb. The PCR product was then directly sequenced using PRISM model 377 DNA sequencer (PE Biosystems, Inc.) to obtain bovine gene sequence (SEQ ID NO: 4, FIGS. 2 and 3). The amino acid sequence deduced from the DNA sequence is shown in FIGS. 2 and 3 as SEQ ID NO: 3. The bovine hypothalamus-derived amino acid sequence (SEQ ID NO: 1) obtained in EXAMPLE 4 corresponds to 2-18 amino acids in SEQ ID NO: 3, and this gene was confirmed to be a gene encoding bovine endogenous ligand gene of GHSR.

EXAMPLE 6 Acquisition of endogenous ligand gene to human GHSR

To acquire a fragment encoding the full-length coding region of human GHSR endogenous ligand gene, the following 2 DNAs were synthesized.

hF : 5'-TGGACCAGCCGTGCAAATCTCTA-3' (SEQ ID NO: 14)

hR : 5'-CCGGTGGCCTAAAGTGTGGTCTCA-3' (SEQ ID NO: 15)

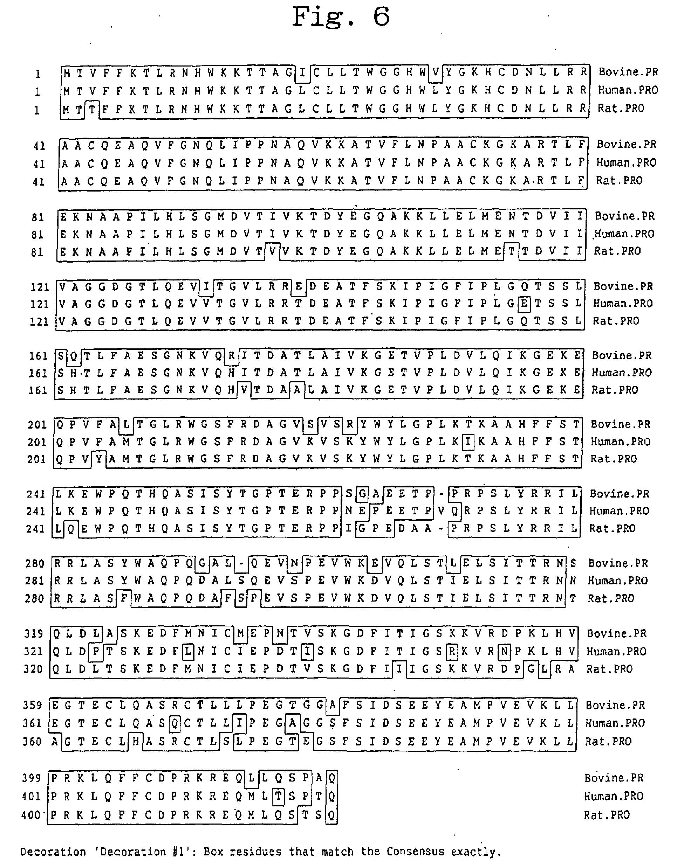

Using Marathon cDNA Amplification Kit (Clontech, Inc.) according to the manual attached, PCR was carried out using as a template cDNA synthesized from 1 µg of human whole brain poly(A)$^+$ RNA. The reaction solution and conditions for PCR were the same as in EXAMPLE 5, except that hF and hR were used as the synthetic DNAs. An aliquot of the PCR product was subjected to electrophoresis to confirm amplification of the PCR product of about 1.3 kb. The PCR product was then directly sequenced using PRISM model 377 DNA sequencer (PE Biosystems, Inc.) to obtain human gene sequence (SEQ ID NO: 6, FIGS. 4 and 5). The amino acid sequence deduced from the DNA sequence is shown in FIGS. 4 and 5 as SEQ ID NO: 5. The gene acquired was confirmed to be human endogenous ligand gene of GHSR, by comparing to the bovine type (FIG. 6).

EXAMPLE 7 Acquisition of endogenous ligand gene to rat GHSR

To acquire a fragment encoding the full-length coding region of rat GHSR endogenous ligand gene, the following 2 DNAs were synthesized.

rF : 5'-AGCGGCGGGCAGCGGAGAAGAT-3' (SEQ ID NO: 16)

rR : 5'-TGACACTGGGCCAGAAGCACAC-3' (SEQ ID NO: 17)

Using Marathon cDNA Amplification Kit (Clontech, Inc.) according to the manual attached, PCR was carried out using as a template cDNA synthesized from 1 µg of rat whole brain poly(A)$^+$ RNA. The reaction solution and conditions for PCR were the same as in EXAMPLE 5, except that rF and rR were used as the synthetic DNAs. An aliquot of the PCR product was subjected to electrophoresis to confirm amplification of the PCR product of about 1.4 kb. The PCR product was then directly sequenced using PRISM model 377 DNA sequencer (PE Biosystems, Inc.) to obtain rat gene sequence (SEQ ID NO: 8, FIGS. 7 and 8). The amino acid sequence deduced from the DNA sequence is shown in FIGS. 7 and 8 as SEQ ID NO: 7. The gene acquired was confirmed to be rat endogenous ligand gene of GHSR, by comparing to the bovine type (FIG. 6).

Industrial Applicability

The DNA encoding the polypeptide of the present invention or the polypeptide of the present invention can be used: 1) for the survey of physiological activities that the polypeptide of the present invention possesses, 2) for preparing synthetic oligonucleotide probes or primers for PCR, 3) for acquiring DNAs encoding ligands to GHSR, 4) for the development of the receptor-binding assay system using the expression system of recombinant receptor protein and screening of a candidate drug, 5) for acquiring antibodies and antisera, 6) for the development of diagnostic agents using DNAs, RNAs, antibodies or antisera, 7) for the development of drugs such as agents for regulating GH secretion (specifically, a prophylactic/therapeutic agent for ① pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, or for ② acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, etc.) or the like, 8) for gene therapy, and the like.

**Claims**

1. A polypeptide containing the same or substantially the same amino acid sequence as the amino acid sequence represented by SEQ ID NO: 1, its amide or ester, or a salt thereof.

2. The polypeptide, its amide or ester, or a salt thereof, according to Claim 1, wherein substantially the same amino acid sequence is the amino acid sequence represented by SEQ ID NO: 10.

3. The polypeptide, its amide or ester, or a salt thereof, according to Claim 1, which contains the same or substantially the same amino acid sequence represented by SEQ ID NO: 3, SEQ ID NO: 5 or SEQ ID NO: 7.

4. A DNA containing a DNA encoding the polypeptide according to Claim 1.

5. The DNA according to Claim 4, which contains the base sequence represented by SEQ ID NO: 2, SEQ ID NO: 4, SEQ ID NO: 6, SEQ ID NO: 8, SEQ ID NO: 9 or SEQ ID NO: 11.

6. An antibody to the polypeptide, its amide or ester, or a salt thereof, according to Claim 1.

7. A diagnostic agent comprising the DNA according to Claim 5 or the antibody according to Claim 6.

8. An antisense DNA having a complementary or substantially complementary base sequence to the DNA according to Claim 5 and capable of suppressing expression of said DNA.

9. An agent comprising the polypeptide or its amide or ester, or a salt thereof, according to Claim 1.

10. A pharmaceutical comprising the polypeptide or its amide or ester, or a salt thereof, according to Claim 1.

11. The pharmaceutical according to Claim 10, which is a prophylactic/therapeutic agent for diseases associated with deficiency of growth hormone.

12. The pharmaceutical according to Claim 10, which is a prophylactic/therapeutic agent for pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome.

**13.** A method for screening a compound or its salt that promotes or inhibits the activity of the polypeptide, its amide or ester, or a salt thereof, according to Claim 1, which comprises using the polypeptide, its amide or ester, or a salt thereof according to Claim 1.

**14.** The method for screening according to Claim 13, which comprises using the polypeptide, its amide or ester, or a salt thereof, according to Claim 1, and a polypeptide containing the same or substantially the same amino acid sequence represented by SEQ ID NO: 18, its amide or ester, or a salt thereof, or a partial peptide thereof, its amide or ester, or a salt thereof.

**15.** A kit for screening a compound or its salt that promotes or inhibits the activity of the polypeptide, its amide or ester, or a salt thereof, according to Claim 1, comprising the polypeptide, its amide or ester, or a salt thereof, according to Claim 1, and a polypeptide containing the same or substantially the same amino acid sequence represented by SEQ ID NO: 18, its amide or ester, or a salt thereof, or a partial peptide thereof, its amide or ester, or a salt thereof.

**16.** A compound or its salt that promotes or inhibits the activity of the polypeptide, its amide or ester, or a salt thereof, according to Claim 1, obtainable using the screening method according to Claim 13 or the screening kit according to Claim 15.

**17.** A growth hormone secretion modulator comprising the compound or its salt according to Claim 16.

**18.** A prophylactic/therapeutic agent for ① pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, or ② acromegaly, TSH-producing tumor, non-secretory (non-functional) pituitary tumor, ectopic ACTH (adrenocorticotropine)-producing tumor, medullary thyroid cancer, VIP-producing tumor, glucagon-producing tumor, gastrin-producing tumor, insulinoma, carcinoid, insulin-dependent or insulin-independent diabetes mellitus, comprising the compound or its salt according to Claim 16.

**19.** Use of the polypeptide, its amide or ester, or a salt thereof, according to Claim 1, for the preparation of a pharmaceutical for the prevention/treatment of pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome.

**20.** A method for preventing/treating pituitary dwarfism, Turner's syndrome, chronic renal failure, chondrodystrophia, adult pituitary deficiency, Down's syndrome, Silver's syndrome, osteogenesis imperfecta or juvenile chronic arthrosis syndrome, which comprises administering the polypeptide, its amide or ester, or a salt thereof, according to Claim 1 to a mammal.

# Fig. 1

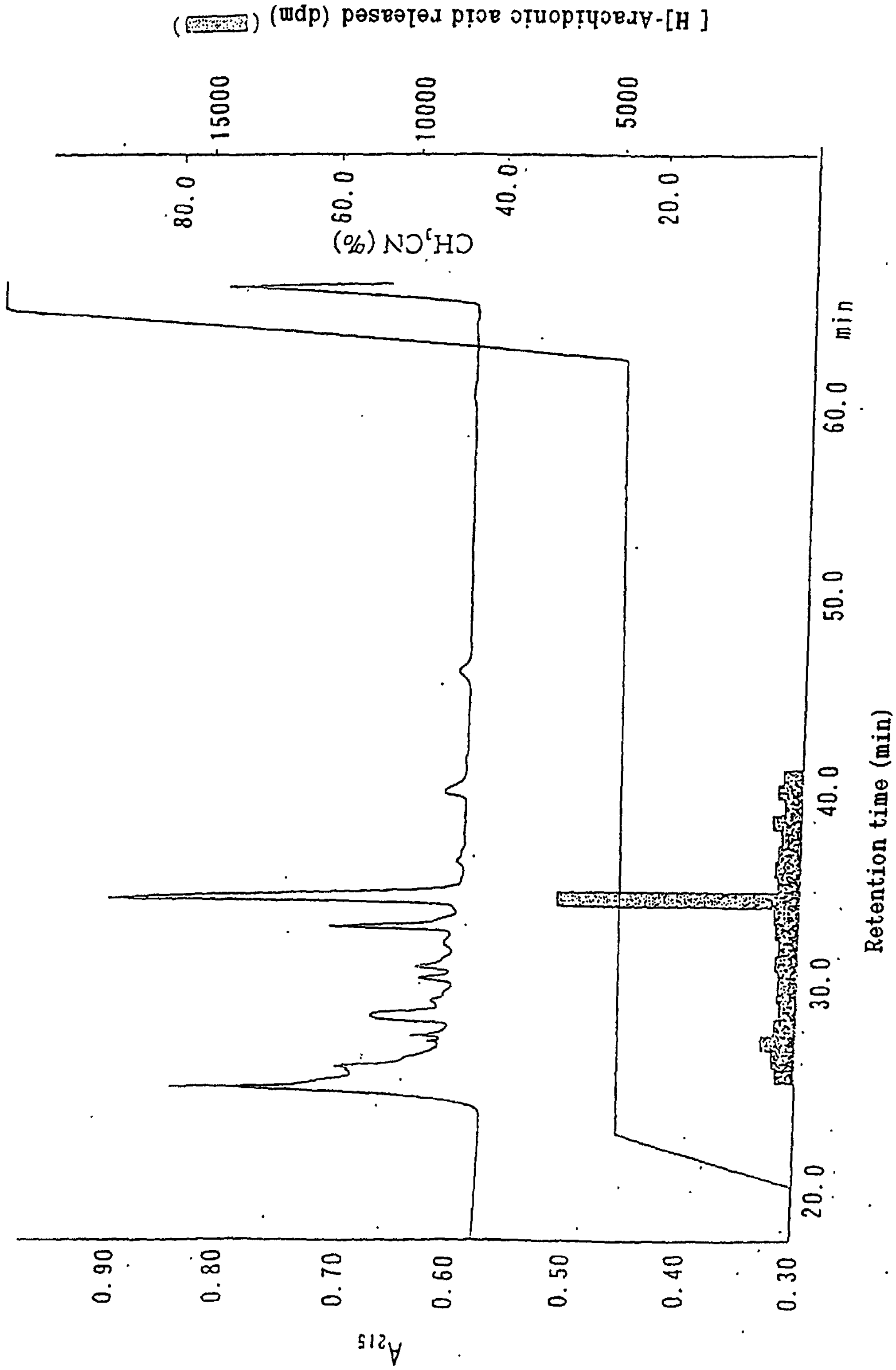
[ H]-Arachidonic acid released (dpm)
15000
10000
5000
80.0
60.0
40.0
20.0
CH3CN (%)
min
20.0
30.0
40.0
50.0
60.0
Retention time (min)
0.90
0.80
0.70
0.60
0.50
0.40
0.30
A215

# Fig. 2

1 ATGACTGTGTTCTTTAAAACGCTTCGAAATCATTGGAAGAAAACGACAGCTGGGATCTGC 60

1 MetThrValPhePheLysThrLeuArgAsnHisTrpLysLysThrThrAlaGlyIleCys 20

61 CTGCTGACATGGGGAGGCCACTGGGTCTATGGCAAACACTGTGATAACCTACTAAGGAGA 120

21 LeuLeuThrTrpGlyGlyHisTrpValTyrGlyLysHisCysAspAsnLeuLeuArgArg 40

121 GCAGCCTGTCAAGAAGCTCAGGTGTTTGGCAATCAACTTATTCCTCCCAATGCGCAAGTG 180

41 AlaAlaCysGlnGluAlaGlnValPheGlyAsnGlnLeuIleProProAsnAlaGlnVal 60

181 AAGAAGGCAACTGTGTTTCTCAACCCTGCAGCTTGCAAAGGCAAAGCCAGGACTCTATTT 240

61 LysLysAlaThrValPheLeuAsnProAlaAlaCysLysGlyLysAlaArgThrLeuPhe 80

241 GAAAAAAATGCTGCCCCGATTTTACACTTATCTGGCATGGATGTTACTATCGTTAAGACA 300

81 GluLysAsnAlaAlaProIleLeuHisLeuSerGlyMetAspValThrIleValLysThr 100

301 GATTATGAGGGCCAGGCCAAGAAACTCCTGGAACTGATGGAAAACACAGATGTGATCATT 360

101 AspTyrGluGlyGlnAlaLysLysLeuLeuGluLeuMetGluAsnThrAspValIleIle 120

361 GTTGCTGGAGGTGACGGGACTTTGCAAGAGGTTATTACTGGAGTTCTTCGAAGAGAAGAT 420

121 ValAlaGlyGlyAspGlyThrLeuGlnGluValIleThrGlyValLeuArgArgGluAsp 140

421 GAGGCTACCTTCAGTAAGATTCCCATCGGATTCATCCCACTGGGCCAGACCAGTAGTTTG 480

141 GluAlaThrPheSerLysIleProIleGlyPheIleProLeuGlyGlnThrSerSerLeu 160

481 AGTCAGACACTCTTTGCCGAAAGTGGAAACAAAGTCCAACGTATTACAGATGCTACACTT 540

161 SerGlnThrLeuPheAlaGluSerGlyAsnLysValGlnArgIleThrAspAlaThrLeu 180

541 GCCATTGTGAAAGGAGAGACGGTTCCACTTGATGTCTTACAGATAAAGGGTGAAAAGGAA 600

181 AlaIleValLysGlyGluThrValProLeuAspValLeuGlnIleLysGlyGluLysGlu 200

601 CAGCCTGTGTTTGCACTCACCGGCCTTCGATGGGGATCCTTCCGAGATGCTGGCGTCTCA 660

201 GlnProValPheAlaLeuThrGlyLeuArgTrpGlySerPheArgAspAlaGlyValSer 220

# Fig. 3

661 GTTAGCAGGTACTGGTATCTTGGGCCTCTAAAAACCAAAGCAGCCCACTTTTTCAGCACT 720

221 ValSerArgTyrTrpTyrLeuGlyProLeuLysThrLysAlaAlaHisPhePheSerThr 240

721 CTTAAGGAGTGGCCTCAGACTCATCAAGCCTCGATCTCGTACACGGGACCTACAGAGAGA 780

241 LeuLysGluTrpProGlnThrHisGlnAlaSerIleSerTyrThrGlyProThrGluArg 260

781 CCTCCCAGTGGAGCAGAAGAGACCCCGCCCCGGCCCTCCTTGTATAGGAGAATATTACGG 840

261 ProProSerGlyAlaGluGluThrProProArgProSerLeuTyrArgArgIleLeuArg 280

841 AGGCTCGCGTCCTACTGGGCACAACCGCAGGGAGCCCTGCAAGAGGTGAACCCAGAGGTC 900

281 ArgLeuAlaSerTyrTrpAlaGlnProGlnGlyAlaLeuGlnGluValAsnProGluVal 300

901 TGGAAGGAAGTGCAGTTGTCCACCCTTGAACTGTCCATCACAACCCGGAACAGTCAGCTT 960

301 TrpLysGluValGlnLeuSerThrLeuGluLeuSerIleThrThrArgAsnSerGlnLeu 320

961 GACCTGGCAAGCAAAGAAGACTTTATGAACATCTGCATGGAACCCAACACCGTCAGCAAA 1020

321 AspLeuAlaSerLysGluAspPheMetAsnIleCysMetGluProAsnThrValSerLys 340

1021 GGAGACTTCATCACTATAGGAAGTAAAAAGGTGAGAGATCCCAAGCTGCATGTTGAGGGC 1080

341 GlyAspPheIleThrIleGlySerLysLysValArgAspProLysLeuHisValGluGly 360

1081 ACCGAGTGTCTCCAAGCCAGCCGCTGCACTCTGCTGCTTCCAGAGGGCACAGGGGGCGCC 1140

361 ThrGluCysLeuGlnAlaSerArgCysThrLeuLeuLeuProGluGlyThrGlyGlyAla 380

1141 TTCAGCATCGACAGTGAGGAGTACGAAGCGATGCCCGTGGAGGTGAAGCTGCTCCCCCGG 1200

381 PheSerIleAspSerGluGluTyrGluAlaMetProValGluValLysLeuLeuProArg 400

1201 AAACTGCAGTTCTTCTGTGACCCTCGCAAGAGGGAGCAGCTGCTGCAGAGCCCGGCCCAG 1260

401 LysLeuGlnPhePheCysAspProArgLysArgGluGlnLeuLeuGlnSerProAlaGln 420

1261 TGA 1263

421 *** 421

# Fig. 4

1 ATGACGGTGTTCTTTAAAACGCTTCGAAATCACTGGAAGAAAACTACAGCTGGGCTCTGC 60

1 MetThrValPhePheLysThrLeuArgAsnHisTrpLysLysThrThrAlaGlyLeuCys 20

61 CTGCTGACCTGGGGAGGCCATTGGCTCTATGGAAAACACTGTGATAACCTCCTAAGGAGA 120

21 LeuLeuThrTrpGlyGlyHisTrpLeuTyrGlyLysHisCysAspAsnLeuLeuArgArg 40

121 GCAGCCTGTCAAGAAGCTCAGGTGTTTGGCAATCAACTCATTCCTCCCAATGCACAAGTG 180

41 AlaAlaCysGlnGluAlaGlnValPheGlyAsnGlnLeuIleProProAsnAlaGlnVal 60

181 AAGAAGGCCACTGTTTTTCTCAATCCTGCAGCTTGCAAAGGAAAAGCCAGGACTCTATTT 240

61 LysLysAlaThrValPheLeuAsnProAlaAlaCysLysGlyLysAlaArgThrLeuPhe 80

241 GAAAAAAATGCTGCCCCGATTTTACATTTATCTGGCATGGATGTGACTATTGTTAAGACA 300

81 GluLysAsnAlaAlaProIleLeuHisLeuSerGlyMetAspValThrIleValLysThr 100

301 GATTATGAGGGACAAGCCAAGAAACTCCTGGAACTGATGGAAAACACGGATGTGATCATT 360

101 AspTyrGluGlyGlnAlaLysLysLeuLeuGluLeuMetGluAsnThrAspValIleIle 120

361 GTTGCAGGAGGAGATGGGACACTGCAGGAGGTTGTTACTGGTGTTCTTCGACGAACAGAT 420

121 ValAlaGlyGlyAspGlyThrLeuGlnGluValValThrGlyValLeuArgArgThrAsp 140

421 GAGGCTACCTTCAGTAAGATTCCCATTGGATTTATCCCACTGGGAGAGACCAGTAGTTTG 480

141 GluAlaThrPheSerLysIleProIleGlyPheIleProLeuGlyGluThrSerSerLeu 160

481 AGTCATACCCTCTTTGCCGAAAGTGGAAACAAAGTCCAACATATTACTGATGCCACACTT 540

161 SerHisThrLeuPheAlaGluSerGlyAsnLysValGlnHisIleThrAspAlaThrLeu 180

541 GCCATTGTGAAAGGAGAGACAGTTCCACTTGATGTCTTGCAGATCAAGGGTGAAAAGGAA 600

181 AlaIleValLysGlyGluThrValProLeuAspValLeuGlnIleLysGlyGluLysGlu 200

601 CAGCCTGTATTTGCAATGACCGGCCTTCGATGGGGATCTTTCAGAGATGCTGGCGTCAAA 660

201 GlnProValPheAlaMetThrGlyLeuArgTrpGlySerPheArgAspAlaGlyValLys 220

# Fig. 5

661 GTTAGCAAGTACTGGTATCTTGGGCCTCTAAAAATCAAAGCAGCCCACTTTTTCAGCACT 720

221 ValSerLysTyrTrpTyrLeuGlyProLeuLysIleLysAlaAlaHisPhePheSerThr 240

721 CTTAAGGAGTGGCCTCAGACTCATCAAGCCTCTATCTCATACACGGGACCTACAGAGAGA 780

241 LeuLysGluTrpProGlnThrHisGlnAlaSerIleSerTyrThrGlyProThrGluArg 260

781 CCTCCCAATGAACCAGAGGAGACCCCTGTACAAAGGCCTTCTTTGTACAGGAGAATATTA 840

261 ProProAsnGluProGluGluThrProValGlnArgProSerLeuTyrArgArgIleLeu 280

841 CGAAGGCTTGCGTCCTACTGGGCACAACCACAGGATGCCCTTTCCCAAGAGGTGAGCCCG 900

281 ArgArgLeuAlaSerTyrTrpAlaGlnProGlnAspAlaLeuSerGlnGluValSerPro 300

901 GAGGTCTGGAAAGATGTGCAGCTGTCCACCATTGAACTGTCCATCACAACACGGAATAAT 960

301 GluValTrpLysAspValGlnLeuSerThrIleGluLeuSerIleThrThrArgAsnAsn 320

961 CAGCTTGACCCGACAAGCAAAGAAGATTTTCTGAATATCTGCATTGAACCTGACACCATC 1020

321 GlnLeuAspProThrSerLysGluAspPheLeuAsnIleCysIleGluProAspThrIle 340

1021 AGCAAAGGAGACTTTATAACTATAGGAAGTCGAAAGGTGAGAAACCCCAAGCTGCACGTG 1080

341 SerLysGlyAspPheIleThrIleGlySerArgLysValArgAsnProLysLeuHisVal 360

1081 GAGGGCACGGAGTGTCTCCAAGCCAGCCAGTGCACTTTGCTTATCCCGGAGGGAGCAGGG 1140

361 GluGlyThrGluCysLeuGlnAlaSerGlnCysThrLeuLeuIleProGluGlyAlaGly 380

1141 GGCTCTTTTAGCATTGACAGTGAGGAGTATGAAGCGATGCCTGTGGAGGTGAAACTGCTC 1200

381 GlySerPheSerIleAspSerGluGluTyrGluAlaMetProValGluValLysLeuLeu 400

1201 CCCAGGAAGCTGCAGTTCTTCTGTGATCCTAGGAAGAGAGAACAGATGCTCACAAGCCCC 1260

401 ProArgLysLeuGlnPhePheCysAspProArgLysArgGluGlnMetLeuThrSerPro 420

1261 ACCCAGTGA 1269

421 ThrGln*** 423

# Fig. 6

```
1    M T V F F K T L R N H W K K T T A G I C L L T W G G H W V Y G K H C D N L L R R   Bovine.PR
1    M T V F F K T L R N H W K K T T A G L C L L T W G G H W L Y G K H C D N L L R R   Human.PRO
1    M T T F F K T L R N H W K K T T A G L C L L T W G G H W L Y G K H C D N L L R R   Rat.PRO

41   A A C Q E A Q V F G N Q L I P P N A Q V K K A T V F L N P A A C K G K A R T L F   Bovine.PR
41   A A C Q E A Q V F G N Q L I P P N A Q V K K A T V F L N P A A C K G K A R T L F   Human.PRO
41   A A C Q E A Q V F G N Q L I P P N A Q V K K A T V F L N P A A C K G K A R T L F   Rat.PRO

81   E K N A A P I L H L S G M D V T I V K T D Y E G Q A K K L L E L M E N T D V I I   Bovine.PR
81   E K N A A P I L H L S G M D V T I V K T D Y E G Q A K K L L E L M E N T D V I I   Human.PRO
81   E K N A A P I L H L S G M D V T V V K T D Y E G Q A K K L L E L M E T T D V I I   Rat.PRO

121  V A G G D G T L Q E V I T G V L R R E D E A T F S K I P I G F I P L G Q T S S L   Bovine.PR
121  V A G G D G T L Q E V V T G V L R R T D E A T F S K I P I G F I P L G E T S S L   Human.PRO
121  V A G G D G T L Q E V V T G V L R R T D E A T F S K I P I G F I P L G Q T S S L   Rat.PRO

161  S Q T L F A E S G N K V Q R I T D A T L A I V K G E T V P L D V L Q I K G E K E   Bovine.PR
161  S H T L F A E S G N K V Q H I T D A T L A I V K G E T V P L D V L Q I K G E K E   Human.PRO
161  S H T L F A E S G N K V Q H V T D A A L A I V K G E T V P L D V L Q I K G E K E   Rat.PRO

201  Q P V F A L T G L R W G S F R D A G V S V S R Y W Y L G P L K T K A A H F F S T   Bovine.PR
201  Q P V F A M T G L R W G S F R D A G V K V S K Y W Y L G P L K I K A A H F F S T   Human.PRO
201  Q P V Y A M T G L R W G S F R D A G V K V S K Y W Y L G P L K T K A A H F F S T   Rat.PRO

241  L K E W P Q T H Q A S I S Y T G P T E R P P S G A E E T P - P R P S L Y R R I L   Bovine.PR
241  L K E W P Q T H Q A S I S Y T G P T E R P P N E P E E T P V Q R P S L Y R R I L   Human.PRO
241  L Q E W P Q T H Q A S I S Y T G P T E R P P I G P E D A A - P R P S L Y R R I L   Rat.PRO

280  R R L A S Y W A Q P Q G A L - Q E V N P E V W K E V Q L S T L E L S I T T R N S   Bovine.PR
281  R R L A S Y W A Q P Q D A L S Q E V S P E V W K D V Q L S T I E L S I T T R N N   Human.PRO
280  R R L A S F W A Q P Q D A F S P E V S P E V W K D V Q L S T I E L S I T T R N T   Rat.PRO

319  Q L D L A S K E D F M N I C M E P N T V S K G D F I T I G S K K V R D P K L H V   Bovine.PR
321  Q L D P T S K E D F L N I C I E P D T I S K G D F I T I G S R K V R N P K L H V   Human.PRO
320  Q L D L T S K E D F M N I C I E P D T V S K G D F I I I G S K K V R D P G L R A   Rat.PRO

359  E G T E C L Q A S R C T L L L P E G T G G A F S I D S E E Y E A M P V E V K L L   Bovine.PR
361  E G T E C L Q A S Q C T L L I P E G A G G S F S I D S E E Y E A M P V E V K L L   Human.PRO
360  A G T E C L H A S R C T L S L P E G T E G S F S I D S E E Y E A M P V E V K L L   Rat.PRO

399  P R K L Q F F C D P R K R E Q L L Q S P A Q                                       Bovine.PR
401  P R K L Q F F C D P R K R E Q M L T S P T Q                                       Human.PRO
400  P R K L Q F F C D P R K R E Q M L Q S T S Q                                       Rat.PRO
```

Decoration 'Decoration #1': Box residues that match the Consensus exactly.

# Fig. 7

1 ATGACCACATTCTTTAAAACACTTCGAAATCATTGGAAGAAAACTACAGCTGGCCTCTGC 60

1 MetThrThrPhePheLysThrLeuArgAsnHisTrpLysLysThrThrAlaGlyLeuCys 20

61 TTGCTGACATGGGGAGGTCACTGGCTCTATGGAAAGCACTGTGATAACCTGCTAAGAAGA 120

21 LeuLeuThrTrpGlyGlyHisTrpLeuTyrGlyLysHisCysAspAsnLeuLeuArgArg 40

121 GCAGCTTGTCAAGAAGCTCAGGTGTTTGGCAACCAGCTCATTCCTCCCAATGCACAAGTG 180

41 AlaAlaCysGlnGluAlaGlnValPheGlyAsnGlnLeuIleProProAsnAlaGlnVal 60

181 AAGAAAGCCACCGTCTTTCTCAATCCTGCAGCTTGCAAAGGCAAAGCCAGAACTCTATTT 240

61 LysLysAlaThrValPheLeuAsnProAlaAlaCysLysGlyLysAlaArgThrLeuPhe 80

241 GAAAAAAATGCTGCCCCAATTTTACACCTGTCTGGCATGGATGTGACTGTCGTCAAGACA 300

81 GluLysAsnAlaAlaProIleLeuHisLeuSerGlyMetAspValThrValValLysThr 100

301 GATTATGAGGGACAAGCCAAGAAGCTCTTGGAGTTGATGGAAACGACCGATGTGATCATT 360

101 AspTyrGluGlyGlnAlaLysLysLeuLeuGluLeuMetGluThrThrAspValIleIle 120

361 GTTGCTGGAGGAGATGGCACCCTGCAGGAGGTTGTTACTGGAGTACTTAGAAGAACAGAT 420

121 ValAlaGlyGlyAspGlyThrLeuGlnGluValValThrGlyValLeuArgArgThrAsp 140

421 GAGGCTACCTTCAGTAAGATTCCAATTGGATTTATCCCGCTGGGACAGACCAGTAGTTTG 480

141 GluAlaThrPheSerLysIleProIleGlyPheIleProLeuGlyGlnThrSerSerLeu 160

481 AGTCACACCCTCTTTGCTGAAAGTGGAAACAAAGTCCAACATGTCACAGATGCCGCACTG 540

161 SerHisThrLeuPheAlaGluSerGlyAsnLysValGlnHisValThrAspAlaAlaLeu 180

541 GCCATTGTGAAAGGGGAGACTGTTCCACTTGACGTCTTGCAGATCAAGGGTGAAAAAGAA 600

181 AlaIleValLysGlyGluThrValProLeuAspValLeuGlnIleLysGlyGluLysGlu 200

601 CAGCCTGTCTATGCCATGACCGGCCTCCGGTGGGGATCCTTCAGAGATGCTGGCGTCAAA 660

201 GlnProValTyrAlaMetThrGlyLeuArgTrpGlySerPheArgAspAlaGlyValLys 220

# Fig. 8

661 GTTAGCAAGTACTGCTATCTTGGTCCTCTAAAAACCAAAGCAGCCCACTTTTTCAGCACT 720

221 ValSerLysTyrTrpTyrLeuGlyProLeuLysThrLysAlaAlaHisPhePheSerThr 240

721 CTTCAGGAGTGGCCTCAGACCCATCAGGCCTCCATCTCTTACACGGGCCCTACAGAGAGA 780

241 LeuGlnGluTrpProGlnThrHisGlnAlaSerIleSerTyrThrGlyProThrGluArg 260

781 CCTCCCATTGGGCCTGAAGATGCTGCTCCCCGGCCTTCTCTGTACAGGAGAATATTACGT 840

261 ProProIleGlyProGluAspAlaAlaProArgProSerLeuTyrArgArgIleLeuArg 280

841 AGGCTTGCCTCATTCTGGGCACAGCCACAGGATGCCTTCTCCCCAGAGGTGAGCCCTGAG 900

281 ArgLeuAlaSerPheTrpAlaGlnProGlnAspAlaPheSerProGluValSerProGlu 300

901 GTCTGGAAAGATGTACAACTGTCCACCATTGAACTGTCCATCACAACCCGAAACACCCAA 960

301 ValTrpLysAspValGlnLeuSerThrIleGluLeuSerIleThrThrArgAsnThrGln 320

961 CTTGACCTGACGAGCAAAGAGGACTTTATGAACATTTGCATTGAGCCTGACACTGTCAGC 1020

321 LeuAspLeuThrSerLysGluAspPheMetAsnIleCysIleGluProAspThrValSer 340

1021 AAAGGAGATTTCATAATCATAGGCAGTAAGAAGGTGAGAGACCCTGGTCTGCGAGCAGCC 1080

341 LysGlyAspPheIleIleIleGlySerLysLysValArgAspProGlyLeuArgAlaAla 360

1081 GGCACCGAGTGTCTCCACGCCAGCCGCTGCACTCTGTCTCTTCCTGAGGGAACTGAGGGC 1140

361 GlyThrGluCysLeuHisAlaSerArgCysThrLeuSerLeuProGluGlyThrGluGly 380

1141 TCCTTCAGCATCGACAGCGAGGAGTATGAAGCCATGCCTGTGGAGGTCAAACTCCTGCCC 1200

381 SerPheSerIleAspSerGluGluTyrGluAlaMetProValGluValLysLeuLeuPro 400

1201 AGAAAACTTCAGTTCTTCTGTGATCCAAGGAAGAGAGAGCAAATGCTGCAGAGCACATCC 1260

401 ArgLysLeuGlnPhePheCysAspProArgLysArgGluGlnMetLeuGlnSerThrSer 420

1261 CAGTGA 1266

421 Gln *** 422

## INTERNATIONAL SEARCH REPORT

International application No.
PCT/JP00/07635

A. CLASSIFICATION OF SUBJECT MATTER
Int.Cl[7] C07K 14/47, C12N 15/12, C07K 16/18, A61K 38/17, G01N 33/15, G01N 33/50

According to International Patent Classification (IPC) or to both national classification and IPC

B. FIELDS SEARCHED

Minimum documentation searched (classification system followed by classification symbols)
Int.Cl[7] C07K 14/47, C12N 15/12, C07K 16/18, A61K 38/17, G01N 33/15, G01N 33/50

Documentation searched other than minimum documentation to the extent that such documents are included in the fields searched

Electronic data base consulted during the international search (name of data base and, where practicable, search terms used)
GenBank/EMBL/DDBJ/GeneSeq, SwissProt/PIR/GeneSeq, MEDLINE (STN)

C. DOCUMENTS CONSIDERED TO BE RELEVANT

| Category* | Citation of document, with indication, where appropriate, of the relevant passages | Relevant to claim No. |
|---|---|---|
| P,X | WO, 00/05365, A1 (SMITHKLINE BEECHAM PLC), 03 February, 2000 (03.02.00) (Family: none) | 1-19 |
| P,X | WO, 00/29422, A1 (HUMAN GENOME SCI INC), 25 May, 2000 (25.05.00) & AU, 200017162, A | 1-19 |
| A | HAWARD, A. D. et al., "A receptor in pituitary and hypothalamus that functions in growth hormone release", Science (1996) Vol.273, No.5277, pp.974-977 | 1-19 |
| P,A | EP, 1033401, A2 (GENSET), 06 September, 2000 (06.09.00) & CA, 2296792, A1 | 1-19 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 December, 2000 (28.12.00) | 16 January, 2001 (16.01.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/07635

**Box I Observations where certain claims were found unsearchable (Continuation of item 1 of first sheet)**

This international search report has not been established in respect of certain claims under Article 17(2)(a) for the following reasons:

1. ☒ Claims Nos.: 20
because they relate to subject matter not required to be searched by this Authority, namely:
Claim 20 pertains to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)